Europäisches Patentamt
European Patent Office
Office européen des brevets

(19)

(11) Publication number: 0 210 065
A1

(12) **EUROPEAN PATENT APPLICATION**

(21) Application number: 86305584.4

(22) Date of filing: 21.07.86

(51) Int. Cl.⁴: C 07 D 499/00
A 61 K 31/43
//C07F7/10, C07F9/65,
C07D405/06, C07D403/06,
C07D413/06, C07D205/08

(30) Priority: 22.07.85 GB 8518421

(43) Date of publication of application:
28.01.87 Bulletin 87/5

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: Coulton, Steven
6 Badgers Close
Horsham West Sussex RH12 4RU(GB)

(72) Inventor: Brooks, Gerald
6 Worcester Road
Reigate Surrey RH2 9HW(GB)

(72) Inventor: Bruton, Gordon
13 Ridgeway Road
Redhill Surrey RH1 6PQ(GB)

(74) Representative: Dayneswood, Trevor et al,
Beecham Pharmaceuticals Patent and Trade Mark
Department Biosciences Research Centre Great Burgh
Yew Tree Bottom Road
Epsom Surrey KT18 5XQ(GB)

(54) **6-Alkylidene penems.**

(57) Compounds of the general formula I:

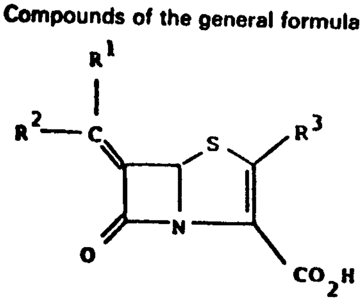

I

and their pharmaceutically acceptable salts and *in vivo* hydrolysable esters,

in which

one of $R^1$ and $R^2$ denotes hydrogen,
the other of $R^1$ and $R^2$ denotes an unsubstituted or substituted non-aromatic heterocyclyl group bonded through a carbon atom thereof, and
$R^3$ denotes hydrogen or an organic group

are novel compounds with β-lactamase inhibitory and antibacterial properties.

EP 0 210 065 A1

NOVEL COMPOUNDS

This invention relates to β-lactam compounds and in particular to a class of 6-alkylidene penems which have β-lactamase inhibitory and antibacterial properties. The compounds are therefore useful in the treatment of antibacterial infections in humans or animals, either alone or in combination with other antibiotics.

European Patent Publication No. EP 0 041 768 A (Beecham; published 16 December 1981; corresponding to U.S. Patent No 4 485 110) discloses 6-alkylidene-2-penems of the general formula (A):

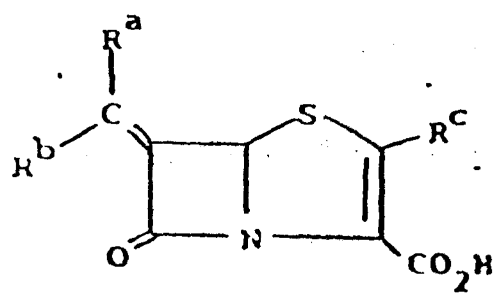

(A)

in which

each of $R^a$ and $R^b$ denotes hydrogen or an optionally substituted hydrocarbon or heterocyclic group, and

$R^c$ denotes hydrogen or an organic group.

Those compounds possess antibacterial activity and also inhibit β-lactamases and have a synergistic effect in combination with other β-lactam antibiotics.

European Patent Publication No. EP 0 120 613 A (Beecham; published 3 October 1984) discloses a sub-group of compounds within the general formula (A) which have better activity than other compounds of the general formula (A). That sub-group consists of compounds of the general formula (B):

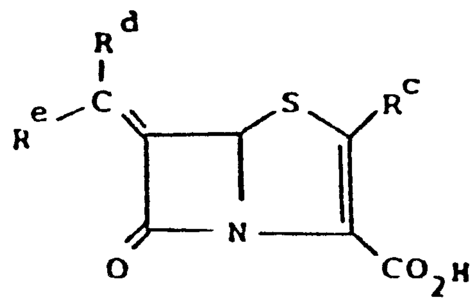

(B)

in which

$R^c$ denotes hydrogen or an organic group;

one of $R^d$ and $R^e$ denotes hydrogen, and

the other of $R^d$ and $R^e$ denotes a group of the sub-formula (C):

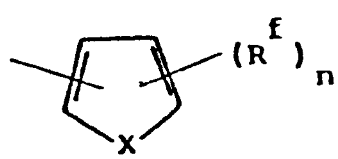

(C)

in which

$R^f$ denotes a substituent group;

X denotes an oxygen atom, a sulphur atom or an >$NR^9$ group;

$R^9$ denotes hydrogen, hydrocarbon or a nitrogen-protecting group; and

n denotes 0, 1, 2 or 3.

According to the present invention there is provided a compound of the general formula I:

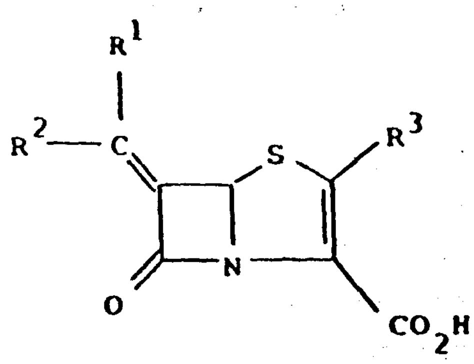

I

or a pharmaceutically acceptable salt or _in vivo_ hydrolysable ester thereof

in which

one of $R^1$ and $R^2$ denotes hydrogen,

the other of $R^1$ and $R^2$ denotes an unsubstituted or substituted non-aromatic heterocyclyl group bonded through a carbon atom thereof, and

$R^3$ denotes hydrogen or an organic group.

The non-aromatic heterocyclyl group denoted by $R^1$ or $R^2$ will have a minimum of three ring atoms, and may suitably have from three to seven ring atoms, advantageously from three to six ring atoms.

At least one of the ring atoms, suitably from one to four of the ring atoms, advantageously from one to three of the ring atoms, preferably one or two of the ring atoms, will be hetero-atoms selected from nitrogen, oxygen and sulphur, the remaining ring atoms being carbon atoms. At least one of the ring atoms, advantageously at least two of the ring atoms will be carbon atoms. The ring may be saturated or unsaturated, provided that the unsaturation does not impart aromaticity. The heterocyclyl group is bonded to the remainder of the molecule through a ring carbon atom.

The maximum possible number of each type of hetero-atom and the various possible combinations of two or more different hetero-atoms, as well as the possible degree of saturation or unsaturation, are dictated by the ring structure and by the requirement for non-aromaticity, and will be apparent to the chemist.

In general, the heterocyclyl ring should advantageously not contain two adjacent oxygen atoms constituting a peroxide structure.

In the case of rings containing unsaturation, such unsaturation may in general be present as a C=C double bond, C=N double bond or N=N double bond.

In the case of rings containing a sulphur atom as a ring hetero-atom, the sulphur atom may be in fully reduced form or may carry one or two oxygen atoms such that the ring is in the form of a sulphoxide or sulphone.

In the case of rings containing a nitrogen atom, the nitrogen atom may be unsubstituted and attached to a

ring double bond, or the nitrogen atom may be substituted by a hydrogen atom or an organic group, or the nitrogen atom may be substituted by an oxygen atom such that the ring nitrogen is in the form of an N-oxide. A substituted nitrogen atom may, for example, be denoted by $-N(R^n)-$, in which $R^n$ denotes a hydrogen atom, an oxygen atom, or an organic group, including, for example, an unsubstituted or substituted hydrocarbon group (for example, alkyl or aryl), an acyl group, a substituted hydroxy group (for example, acyloxy or alkoxy), a substituted amino group (for example, acylamino), or a nitrogen-protecting group (which may optionally be removed at any convenient stage.)

The non-aromatic heterocyclyl group $R^1$ or $R^2$ may be unsubstituted or may be substituted by one or more substituents bonded to the ring at a carbon atom.

Suitably, the heterocyclyl group may be unsubstituted or may carry one or two substituents.

Examples of suitable substituents which may be present on a carbon atom in the heterocyclyl group $R^1$ or $R^2$ include $(C_{1-6})$alkanoyl, $(C_{1-6})$alkanoyloxy, heterocyclyl, amino, $(C_{1-6})$alkanoylamino, (mono or di)-$(C_{1-6})$alkylamino, hydroxy, $(C_{1-6})$alkoxy, sulpho, mercapto, $(C_{1-6})$alkylthio, $(C_{1-6})$alkylsulphinyl, $(C_{1-6})$alkyl-sulphonyl, heterocyclylthio, arylthio, sulphamoyl, carbamoyl, amidino, guanidino, hydroxy, cyano, nitro, halogen, carboxy, carboxy salts, carboxy esters, arylcarbonyl, and heterocyclylcarbonyl groups, and also unsubstituted or substituted $(C_{1-6})$alkyl, $(C_{2-6})$alkenyl, $(C_{2-6})$alkynyl, aryl, and aryl$(C_{1-6})$alkyl groups.

Examples of suitable optional substituents for the above-mentioned $(C_{1-6})$alkyl, $(C_{2-6})$alkenyl, $(C_{2-6})$alkynyl, aryl and aryl$(C_{1-6})$alkyl substituents include $(C_{1-6})$alkanoyl, $(C_{1-6})$alkanoyloxy, heterocyclyl, amino, $(C_{1-6})$alkanoylamino, (mono or di)-$(C_{1-6})$alkylamino, hydroxy, $(C_{1-6})$alkylsulphinyl, $(C_{1-6})$alkylsulphonyl, heterocyclylthio, arylthio, sulphamoyl, carbamoyl, amidino, guanidino, nitro, halogen, carboxy, carboxy salts, carboxy esters, arylcarbonyl and heterocyclylcarbonyl groups.

In addition to or instead of the above-mentioned monovalent substituents for the heterocyclyl group $R^1$ or $R^2$, the heterocyclyl group $R^1$ or $R^2$ may optionally contain one or more divalent substituents, such as those of the formulae =O, =S, =N($R^n$), and =$CR^{01}R^{02}$, in which $R^n$ is defined as above, and each of $R^{01}$ and $R^{02}$, which may be identical or different, denotes a hydrogen atom, an unsubstituted or substituted hydrocarbon group, or an unsubstituted or substituted heterocyclyl group. Such a divalent substituent should not, of course, impart aromaticity to the ring.

The heterocyclyl group $R^1$ or $R^2$ may be disubstituted on a single ring carbon atom by two of the above-mentioned monovalent substituents, which may be identical or different. Alternatively, it may be disubstituted on a single carbon atom by a ring in such a manner as to form a spirocyclic ring system.

The heterocyclyl group $R^1$ or $R^2$ may contain a monovalent substituent (for example those listed above) on the carbon atom through which the group is bonded to the remainder of the molecule. Such a substituent is advantageously a small substituent, for example a methyl group.

When the heterocyclyl group $R^1$ or $R^2$ includes a carboxy salt or carboxy ester substituent, that substituent is suitably a pharmaceutically acceptable salt or pharmaceutically acceptable ester.

When the heterocyclyl group $R^1$ or $R^2$ is or includes a basic moiety, the compound according to the invention may exist in zwitterionic form.

The term 'heterocyclyl' as used herein (except in defining the group $R^1$ or $R^2$) includes aromatic and non-aromatic, single and fused, rings containing up to four hetero-atoms in each ring selected from oxygen, nitrogen and sulphur, which rings may be unsubstituted or substituted by up to three groups selected from halogen, $(C_{1-6})$alkyl, $(C_{1-6})$alkoxy, halo$(C_{1-6})$alkyl, hydroxy, amino, carboxy, $(C_{1-6})$alkoxycarbonyl, $(C_{1-6})$alkoxycarbonyl$(C_{1-6})$alkyl, aryl, $(C_{1-6})$alkylthio, arylthio, mercapto and oxo groups.

The term 'aryl' as used herein includes phenyl and naphthyl, which may be unsubstituted or substituted by up to five, preferably up to three, groups selected from halogen, $(C_{1-6})$alkyl, phenyl, $(C_{1-6})$alkoxy, halo$(C_{1-6})$alkyl, hydroxy, amino, nitro, carboxy, $(C_{1-6})$alkoxycarbonyl, $(C_{1-6})$alkoxycarbonyl$(C_{1-6})$alkyl, $(C_{1-6})$alkylcarbonyloxy, $(C_{1-6})$alkylcarbonyl, $(C_{1-6})$alkylthio, arylthio, and mercapto groups.

The term 'hydrocarbon' as used herein includes groups having up to 18 carbon atoms, suitably up to 10 carbon atoms, conveniently up to 6 carbon atoms. Suitable hydrocarbon groups include $(C_{1-6})$alkyl, $(C_{2-6})$alkenyl, $(C_{2-6})$alkynyl, $(C_{3-7})$cycloalkyl, $(C_{3-7})$cycloalkyl-$(C_{1-6})$alkyl, aryl, and aryl$(C_{1-6})$alkyl.

Examples of suitable optional substituents for the above-mentioned hydrocarbon groups include $(C_{1-6})$alkyl, $(C_{2-6})$alkenyl, $(C_{2-6})$alkynyl, aryl, aryl$(C_{1-6})$alkyl $(C_{1-6})$alkanoyl, $(C_{1-6})$alkanoyloxy, heterocyclyl, amino, $(C_{1-6})$alkanoylamino, (mono or di)-$(C_{1-6})$alkylamino, hydroxy, $(C_{1-6})$alkylsulphinyl, $(C_{1-6})$alkylsulphonyl, heterocyclylthio, arylthio, sulphamoyl, carbamoyl, amidino, guanidino, cyano, nitro, halogen, carboxy, carboxy salts, carboxy esters, arylcarbonyl and heterocyclylcarbonyl groups.

The term 'acyl' as used herein means 'carboxylic acid acyl', for example unsubstituted or substituted hydrocarbon-carbonyl, for example alkanoyl or arylcarbonyl.

The non-aromatic heterocyclyl group denoted by $R^1$ or $R^2$ may suitably be a three-membered ring, advantageously containing only one hetero-atom in the ring, and advantageously being saturated. The ring may suitably contain up to three monovalent substituents, which may advantageously be selected from unsubstituted or substituted alkyl, aryl, acyl and heterocyclyl groups. The ring may alternatively contain a divalent substituent or be disubstituted by a ring on a single carbon atom to form a spirocyclic ring system. Examples of suitable heterocyclyl groups include those of the general formula (in which possible substituents have been omitted):

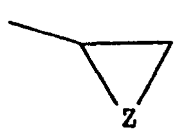

in which

Z denotes O, S, or N($R^n$); and

$R^n$ is defined as above.

Particular examples of three-membered non-aromatic heterocyclyl groups $R^1$ or $R^2$ include oxiranyl groups, optionally substituted in the 3-position by one of the above-listed monovalent substituents for heterocyclyl groups $R^1$ or $R^2$, which may suitably be, for example, a $(C_{1-6})$alkyl group, e.g. a methyl group. Such heterocyclyl groups may be represented by the formula

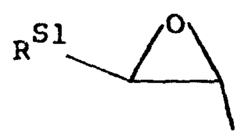

in which $R^{S1}$ donotes a hydrogen atom or one of the said above-listed monovalent substituents.

The non-aromatic heterocyclyl group denoted by $R^1$ or $R^2$ may suitably be a four-membered ring, advantageously containing one or two heteroatoms in the ring (with the proviso that the ring does not contain two adjacent oxygen atoms), and advantageously being saturated or containing one double bond. The ring may suitably contain up to 5 monovalent substituents, which may

advantageously be selected from those listed previously as suitable substituents in the non-aromatic heterocyclyl group $R^1$ or $R^2$. The ring may alternatively contain one or two divalent substituents or be disubstituted by a ring on a single carbon atom to form a spirocyclic ring system. Any unsaturation or second heteroatom in the ring will, of course, reduce the possible number of substituents. Examples of suitable heterocyclyl groups include the following (in which possible substituents have been omitted):

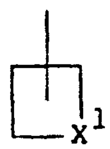
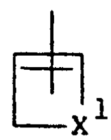
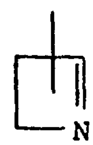

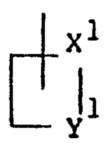
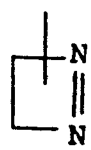
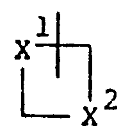

in which

the bond shown centrally to the ring may be attached at any available ring carbon atom;

each of $X^1$ and $X^2$, which may be identical or different, denotes O, S, SO, $SO_2$, NO, or $N(R^n)$;

$Y^1$ denotes S, SO, $SO_2$, NO, or $N(R^n)$; and

$R^n$ is defined as above.

Particular examples of four-membered non-aromatic heterocyclyl groups $R^1$ or $R^2$ include azetidinyl groups, especially azetidin-2-yl groups, optionally substituted

by one or two of the above-listed monovalent substituents for heterocyclyl groups $R^1$ or $R^2$ (for example, ($C_{1-6}$)alkyl groups, e.g. methyl groups) or by one of the above-listed divalent substituents for such groups (for example, oxo groups). Such heterocyclyl groups may be represented by the formula:

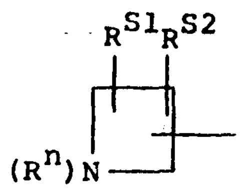

in which

$R^n$ is defined as above, and

each of $R^{S1}$ and $R^{S2}$ denotes a hydrogen atom or one of the said above-listed monovalent substituents, or

$R^{S1}$ and $R^{S2}$ together denote one of the said above-listed divalent substituents.

The non-aromatic heterocyclyl group denoted by $R^1$ or $R^2$ may suitably be a five-membered ring, advantageously containing one, two or three heteroatoms in the ring (with the proviso that the ring does not contain two adjacent oxygen atoms), and advantageously being saturated or containing one double bond. The ring may suitably contain up to seven monovalent substituents, which may advantageously be selected from those listed previously as suitable substituents in the non-aromatic heterocyclyl group $R^1$ or $R^2$. The ring may alternatively contain up to three divalent substituents, with the proviso that the divalent

substituent(s) should not impart aromaticity to the ring. The ring may furthermore be disubstituted by a ring on a single carbon atom to form a spirocyclic ring system. Any unsaturation or second or third heteroatom in the ring, will of course, reduce the possible number of substituents. Examples of suitable heterocyclyl groups include the following (in which possible substituents have been omitted):

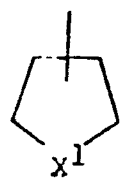
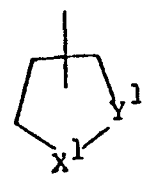
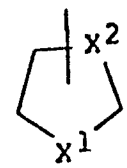

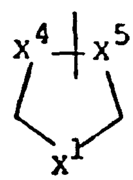
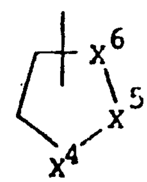

in which

the bond shown centrally to the ring may be attached at any available ring carbon atom;

each of $X^1$, $X^2$, $X^4$, $X^5$ and $X^6$, which may be identical or different, denotes O, S, SO, $SO_2$, NO, or $N(R^n)$, with the proviso that $X^4$ and $X^5$, or $X^5$ and $X^6$, do not simultaneously both denote O; and

$R^n$ is defined as above;

as well as corresponding unsaturated groups containing one double bond in the ring, which may be a C=C, C=N, or N=N bond.

Particular examples of five-membered non-aromatic heterocyclyl groups $R^1$ or $R^2$ include pyrrolidinyl, (di or tetra)hydro-oxazolyl or -isoxazolyl, and dioxolanyl groups, each of which may optionally be substituted by one or two of the above-listed monovalent substituents for heterocyclyl groups $R^1$ or $R^2$ (for example, $(C_{1-6})$alkyl groups, e.g. methyl groups) or by one of the above-listed divalent substituents for such groups (for example, oxo groups).

The non-aromatic heterocyclyl group denoted by $R^1$ or $R^2$ may suitably be a six-membered ring, advantageously containing from one to four heteroatoms in the ring (with the proviso that the ring does not contain two adjacent oxygen atoms), and advantageously being saturated or containing one or two double bonds. The ring may suitably contain up to nine monovalent substituents, which may advantageously be selected from those listed previously as suitable subsitutents in the non-aromatic heterocyclyl group $R^1$ or $R^2$. The ring may alternatively contain up to three divalent substituents with the proviso that it or they should not impart aromaticity to the ring. The ring may furthermore be disubstituted by a ring on a single carbon atom to form a spirocyclic ring system. Any unsaturation or additional heteroatom in the ring will, of course, reduce the possible number of substituents. Examples of suitable heterocyclyl groups include the following (in which possible substituents have been omitted):

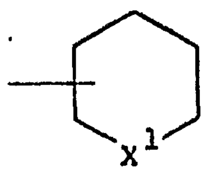
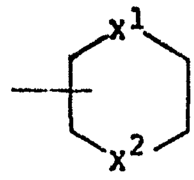
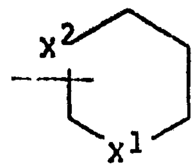
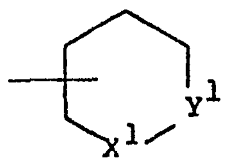
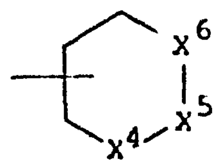
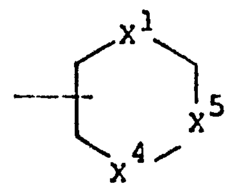
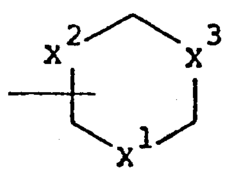

in which

the bond shown centrally to the ring may be attached at any available ring carbon atom;

each of $X^1$, $X^2$, $X^3$, $X^4$, $X^5$ and $X^6$, which may be identical or different, denotes O, S, SO, $SO_2$, NO, or $N(R^n)$, with the proviso that $X^4$ and $X^5$, or $X^5$ and $X^6$, do not simultaneously both denote O; and

$Y^1$ and $R^n$ are defined as above;

as well as corresponding unsaturated groups containing one or two double bonds in the ring, each of which double bonds may be a C=C, C=N, or N=N bond.

Particular examples of six-membered non-aromatic heterocyclyl groups $R^1$ or $R^2$ include (di or tetra) hydropyranyl groups, more particularly -pyran-2-yl groups, and dihydropyranyl groups, which may optionally be substituted by one or two of the above-listed monovalent substituents for heterocyclyl groups $R^1$ or $R^2$ (for example, $(C_{1-6})$alkyl groups, e.g. methyl groups) and/or by one of the above-listed divalent substituents for such groups (for example, oxo groups).

In general formula (I), $R^3$ represents hydrogen or an organic group, which may suitably be linked through a sulphur or carbon atom. For example, $R^3$ may represent hydrogen or a group of formula $-R^4$ or $-SR^4$, where $R^4$ denotes an unsubstituted or substituted $(C_{1-10})$hydrocarbon or heterocyclyl group. Preferably, $R^3$ represents hydrogen, $(C_{1-10})$alkyl or $(C_{1-10})$alkylthio, or substituted $(C_{1-10})$alkyl or substituted $(C_{1-10})$-alkylthio, wherein the substituent may be hydroxy, $(C_{1-6})$alkoxy, $(C_{1-6})$alkanoyloxy, halogen, mercapto, $(C_{1-6})$alkylthio, heterocyclylthio, amino, (mono or di)-$(C_{1-6})$alkylamino, $(C_{1-6})$alkanoylamino, carboxy, or $(C_{1-6})$alkoxycarbonyl.

Examples of suitable organic groups $R^3$ include methyl, ethyl, propyl, methylthio, ethylthio, methylsulphinyl, ethylsulphinyl, hydroxymethyl, methoxymethyl, ethoxymethyl, acetoxymethyl, (1 or 2)-acetoxyethyl, aminomethyl, 2-aminoethyl, acetamidomethyl, 2-acetamidoethyl, carboxymethyl, 2-hydroxyethylthio, methoxymethylthio, 2-methoxyethylthio, acetoxymethylthio, 2-aminoethylthio, acetamidomethylthio, 2-acetamidoethylthio, carboxymethylthio, 2-carboxyethylthio, aryl (especially phenyl), arylthio (especially phenylthio), pyridyl, pyrimidyl,

isoxazolyl, pyrimidylthio, tetrazolylthio, and pyridylthio groups. In particular, $R^3$ may be hydrogen.

Alternatively, $R^3$ may denote a group of the formula $-OR^7$ or $-R^8$, in which $R^7$ denotes an unsubstituted or substituted phenyl, naphthyl, thienyl, pyridyl, quinolyl or isoquinolyl group, and $R^8$ denotes a nitrogen-containing heterocyclyl ring bonded through a ring-nitrogen atom. Examples of groups denoted by $R^7$ and $R^8$, and also examples of suitable substituents for the aryl or heterocyclyl rings denoted by $R^7$, are given in GB 2 102 798 A (Hoechst), EP 0 099 059 A (Hoechst), EP 0 148 128 A (Ciba-Geigy; corresponding to AU 84/37236).

Pharmaceutically acceptable in vivo hydrolysable esters (also referred to as 'metabolisable esters') of the compounds of the general formula I are those esters which hydrolyse in the human body to produce the parent acid or its salt. Such esters may be identified by oral or intravenous administration to a test animal, and subsequent examination of the test animal's body fluids for the presence of the compound of the formula I or a salt thereof.

In some cases, the in vivo hydrolysable ester moiety may constitute a link between two different active ingredient moieties, one of which is a compound according to the invention and the other of which may be another therapeutically active compound, such that on in vivo hydrolysis of the ester moiety, the ester link breaks to give the two separate active compounds. The linked entity may be referred to as a 'mutual pro-drug'.

Suitable <u>in vivo</u> hydrolysable ester groups include those of part-formulae (a), (b) and (c):

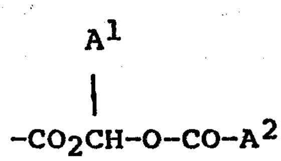

(a)

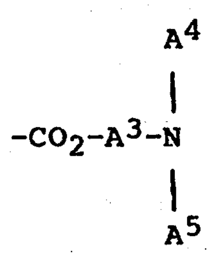

(b)

$-CO_2CH_2-OA^6$ (c)

in which

$A^1$ denotes hydrogen, methyl, or phenyl;

$A^2$ denotes $(C_{1-6})$alkyl, $(C_{1-6})$alkoxy or phenyl; or

$A^1$ and $A^2$ together denote 1,2-phenylene, which may be unsubstituted or substituted by one or two methoxy groups;

$A^3$ denotes $(C_{1-6})$alkylene, which may be unsubstituted or substituted by a methyl or ethyl group;

each of $A^4$ and $A^5$ which may be identical or different, denotes $(C_{1-6})$alkyl; and

$A^6$ denotes $(C_{1-6})$alkyl.

Examples of suitable *in vivo* hydrolysable ester groups include acetoxymethyl, pivaloyloxymethyl, α-acetoxyethyl, α-acetoxybenzyl, α-pivaloyloxyethyl, ethoxycarbonyloxymethyl, α-ethoxycarbonyloxyethyl, dimethylaminomethyl, diethylaminomethyl, phthalidyl and dimethoxyphthalidyl groups.

Suitable pharmaceutically acceptable salts of the 3-carboxylic acid group of the compound of formula I include metal salts, e.g. aluminium salts, alkali metal salts (e.g. sodium or potassium salts), alkaline earth metal salts (e.g. calcium or magnesium salts), ammonium salts, and substituted ammonium salts, for example those with lower alkylamines (e.g.triethylamine), hydroxy-lower alkylamines (e.g. 2-hydroxyethylamine), di(2-hydroxyethyl)amine or tri(2-hydroxyethyl)amine), cycloalkylamines (e.g. dicyclohexylamine), or with procaine, and also dibenzylamine, N,N-dibenzylethylenediamine, 1-ephenamine, N-ethylpiperidine, N-benzyl-β-phenethylamine, dehydroabietylamine, N,N'-bishydroabietylethylene-diamine, bases of the pyridine type (e.g. pyridine, collidine and quinoline), and other amines which have been or can be used to form salts with penicillins.

The compounds of the general formula I and their salts may exist in hydrated or non-hydrated form.

The compounds of the general formula I and also the salts and esters thereof may exist in two optically active forms with respect to the 5-position of the penem ring system, and it is to be understood that both such forms as well as racemic mixtures thereof are embraced by the present invention. It is believed that the more active form is that of structure IA:

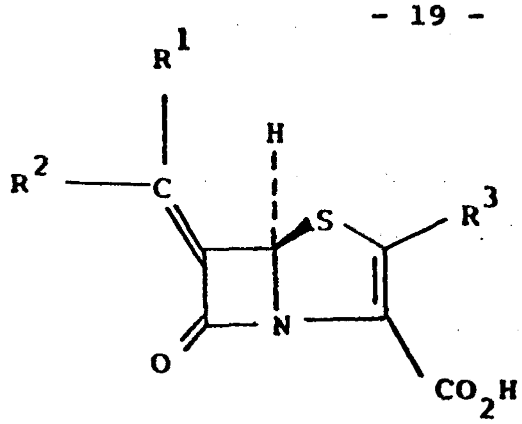

IA

in which $R^1$, $R^2$ and $R^3$ are defined as above.

The compounds of the general formula I and also the salts and esters thereof may also possess a chiral (asymmetric) centre at the ring carbon atom by which the heterocyclic ring is attached to the remainder of the molecule. In some cases, the compounds of the invention will also contain chiral (asymmetric) centres at other positions within the heterocyclic ring. It is to be understood that all such diasteriomeric forms, as well as all other isomeric forms, whether optical or geometric isomers, and mixtures of any or all such isomers are embraced by the present invention.

In general formulae I and IA, it is thought to be advantageous that $R^1$ denotes the heterocyclyl group and that $R^2$ denotes a hydrogen atom.

Examples of individual compounds according to the invention include:

(5R) (Z)-6-[(1,3-dioxolan-4-yl)methylene]penem-3-carboxylic acid;

(5RS) (Z)-6-(2,3-epoxybutylidene)penem-3-carboxylic acid;

(5R) (Z)-6-(2,3-epoxybutylidene)penem-3-carboxylic acid;

(5RS) (Z)-6-[(N-benzyloxycarbonyl-pyrrolidin-2-yl) methylene]penem-3-carboxylic acid;

(5R) (Z)-6-([(2S)-azetidin-2-yl]methylene)penem-3-carboxylic acid;

(5RS) (Z)-6-[(3,4-dihydro-2H-pyran-2-yl)methylene]-penem-3-carboxylic acid;

(5R) (E)-6-([(4R)-1-methyloxy-2-oxoazetidin-4-yl]-methylene)penem-3-carboxylic acid;

(5R) (Z)-6-[(3-methyl-2-isoxazolin-5-yl)methylene]-penem-3-carboxylic acid;

(5RS) (Z)-6-[(1-methyl-2-oxo-pyrrolidin-5-yl)-methylene]penem-3-carboxylic acid;

as well as pharmaceutically acceptable salts and in-vivo hydrolysable esters thereof.

A compound of the general formula I, or a salt or ester thereof, may be prepared by eliminating the elements of a compound of the general formula XIII:

$$H-X^{o} \qquad XIII$$

from a penem or penem intermediate of the general part-formula XIV:

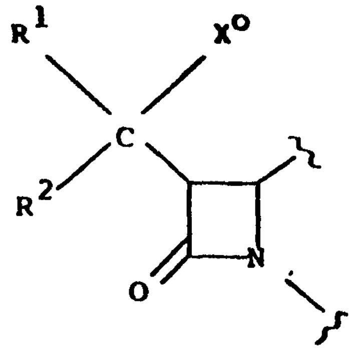

XIV

in which

$R^1$ and $R^2$ are defined as above, and

$X^o$ denotes a hydroxy group or a leaving group,

to give a compound of the general part-formula XV:

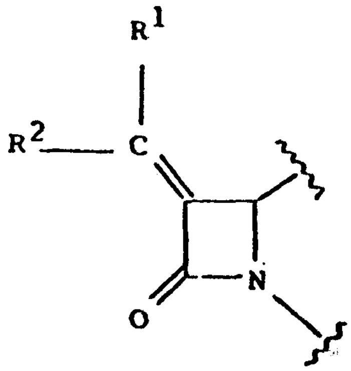

XV

in which $R^1$ and $R^2$ are defined as above,

and, if the resulting compound of the general formula XV is a penem intermediate, converting it into a penem of the general formula I or a salt or ester thereof.

The compound of the general part-formula XIV may suitably be a compound of the general part-formula XIVA:

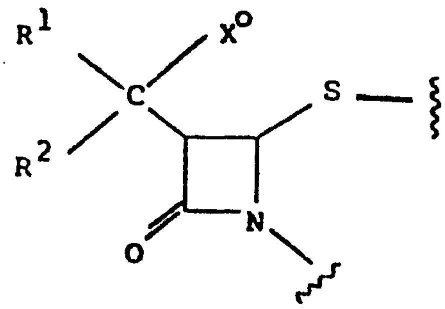

XIVA

in which $R^1$, $R^2$ and $X^o$ are defined as above. More especially it may be a compound of the general formula XIVB:

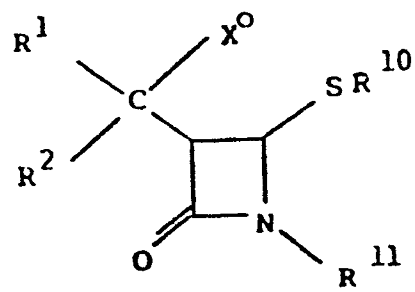

XIVB

in which

$R^1$, $R^2$ and $X^o$ are defined as above,

$R^{10}$ denotes $(C_{1-6})$alkyl, aryl, aryl$(C_{1-6})$alkyl, $(C_{1-6})$alkylthio, arylthio, hetero-aromatic-thio, acyl (for example, $(C_{1-6})$alkylcarbonyl, especially acetyl), $(C_{2-6})$alkenyl (especially vinyl), or aryl$(C_{2-6})$alkenyl, all of which may optionally be substituted, and

$R^{11}$ denotes hydrogen or an N-protecting group, or

$R^{10}$ and $R^{11}$ together denote the remainder of a penem nucleus, which may be substituted and/or may optionally carry a protecting group.

In the case where $R^{10}$ and $R^{11}$ in general formula XIVB together denote the remainder of a penem nucleus, they may suitably together denote the sub-formula XVI:

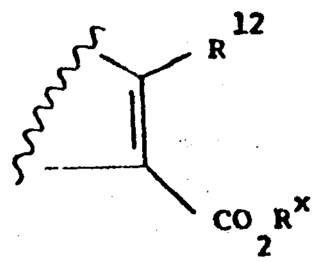

XVI

in which

$R^{12}$ denotes the hydrogen atom or organic group $R^3$ or a group convertible into $R^3$ during the preparation of a penem of the general formula I or salt or ester thereof, and

$R^x$ denotes hydrogen or a carboxyl-blocking group.

In that case, the penem or penem intermediate of the general part-formula XIV is of the general formula XIVD given below.

A carboxyl-blocking group $R^x$ (also referred to as a carboxyl-protecting group) is suitably a group that can readily be removed at a later stage of the penem preparation process.

Examples of suitable carboxyl-blocking derivatives that may form the group $-CO_2R^x$ include salt, ester, and anhydride derivatives of the carboxylic acid.

The salts may be organic or inorganic and need not be pharmaceutically acceptable. Examples of suitable salt-forming groups $R^x$ include inorganic salts, for example alkali metal atoms (e.g. lithium and sodium), other metal atoms, tertiary amino groups (e.g. tri-lower-alkylamino, N-ethylpiperidino, and dimethylpiperazino groups). A preferred salt-forming group $R^x$ is the triethylamino group.

An ester-forming group $R^x$ is advantageously one that can be removed under conventional conditions. Examples of suitable ester-forming groups $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluene-sulphonylethyl, and methoxymethyl groups, and also silyl, stannyl and phosphorus-containing groups, and oxime radicals of formula $-N=CHR^o$ in which $R^o$ denotes aryl or heterocyclyl. Furthermore, the ester-forming group $R^x$ may be an in vivo hydrolysable ester group including, in particular, those listed above.

The free carboxyl group may be regenerated from any of the above esters by usual methods appropriate to the particular $R^x$ group, for example, by acid-catalysed, base-catalysed or enzymically-catalysed hydrolysis, or by hydrogenolysis. The hydrolysis must of course be carried out under conditions in which the groups on the rest of the molecule are stable.

When it is desired to produce a compound of the general formula I in the form of a free acid or in the form of a salt, by a process according to the invention, it is generally advantageous to use a compound in which $R^x$ denotes a carboxyl-blocking group. When it is desired to produce a compound of the general formula I in the form of a pharmaceutically acceptable ester, it is generally convenient to use a compound in which $R^x$ denotes the desired ester group.

The process step according to the invention involves the elimination of the elements of a compound $H\text{-}X^o$ from a penem or penem intermediate of the general part-formula XIV, in which $X^o$ denotes a hydroxy group or a leaving group.

In the case where $X^o$ denotes a hydroxy group, the compound of the formula $H\text{-}X^o$ being eliminated is water and the elimination reaction is a dehydration reaction, which may suitably be carried out by treating a compound of the general part-formula XIV with a compound of the general formula XVII:

$$R^{13}O_2C\text{-}N\text{=}N\text{-}CO_2R^{14} \qquad \text{XVII}$$

in which each of $R^{13}$ and $R^{14}$, which may be identical or different, denotes aryl, $(C_{1-6})$alkyl or aryl$(C_{1-6})$alkyl,

and a with compound of the general formula XVIII:

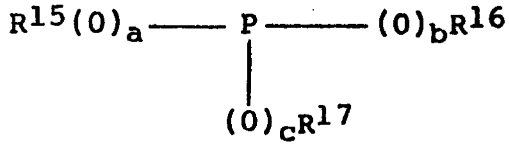

XVIII

in which

each of <u>a</u>, <u>b</u> and <u>c</u> which may be identical or different, denotes 0 or 1, and

each of $R^{15}$, $R^{16}$ and $R^{17}$, which may be identical or different, denotes aryl, $(C_{1-6})$alkyl or aryl$(C_{1-6})$alkyl.

In the compounds of the general formula VI, $R^{13}$ and $R^{14}$ are preferably selected from methyl, ethyl, propyl, butyl, phenyl, and benzyl, the ethyl and isopropyl groups being preferred. Advantageously, $R^{13}$ and $R^{14}$ may be identical. A preferred compound of the general formula XVIII is diethyl azodicarboxylate.

Preferred compounds of the general formula XVIII include triarylphosphines and trialkylphosphites. Preferred groups $R^{15}$, $R^{16}$ and $R^{17}$ include methyl, ethyl, n-propyl, n-butyl, benzyl, phenyl and methoxyphenyl. Advantageously, $R^{15}$, $R^{16}$ and $R^{17}$ are all identical. A preferred compound of the general formula XVIII is triphenylphosphine.

Advantageously, approximately two equivalents of each the compounds of the general formulae XVII and XVIII are used per mole of the compound of the general part-formula XIV.

The dehydration reaction may suitably be carried out at a non-extreme temperature, for example a temperature of from -20°C to +100°C. It may be convenient to begin the reaction at a depressed temperature, for example 0°C, and then to allow the temperature to rise to about room temperature.

The reaction may suitably be carried out in an inert aprotic organic solvent. Suitable solvents include tetrahydrofuran, dioxane, ethyl acetate, benzene, and dichloromethane.

In the cases where $X^o$, in general formula XIV, denotes a leaving group, which will hereinafter be referred to as $X^{01}$, it may suitably be a halogen atom or a group of one of the formulae

$-O-SO_2-(O)_n-R^{18}$ XIXA

$-O-CO-(O)_n-R^{18}$ XIXB or

$-O-PO-(OR^{19})_2$ XIXC

in which

n denotes 0 or 1,

$R^{18}$ denotes $(C_{1-6})$alkyl, aryl or aryl$(C_{1-6})$alkyl,

and

$R^{19}$ denotes $(C_{1-6})$alkyl or aryl.

Preferred groups of formula XIXB are those in which n denotes zero and $R^{18}$ denotes $(C_{1-6})$alkyl, especially the acetoxy group.

The elimination of the elements of a compound of the general formula XIII in which $X^o$ denotes a leaving group $X^{01}$ from a compound of the general formula XIV, may suitably be effected by treating the compound of the general formula XIV with a base in an aprotic medium.

Suitable bases for that purpose include, for example, powdered inorganic bases, for example alkali metal carbonates, bicarbonates, hydroxides, and hydrides (e.g. powdered potassium carbonate), and also organic bases of low nucleophilicity, for example 1,8-diazabicyclo[5.4.0]undec-7-ene. Suitable solvents for use as the aprotic medium in this reaction include, for example, dimethylformamide, hexamethylphosphoramide, dichloromethane, and tetrahydrofuran.

The elimination may suitably be effected at a low temperature, for example a temperature of from -70°C to +70°C, advantageously from -40°C to 0°C.

The compounds of the general formula XIV in which $X^o$ denotes a leaving group $X^{01}$ may suitably be prepared from the corresponding compound in which $X^o$ denotes a hydroxy group by converting the hydroxy group to a leaving group $X^{01}$. Alternatively, in the case of the compounds of the general formula XIVD below, the leaving group $X^{01}$ may be introduced into the molecule at an earlier stage in the synthesis of the penem nucleus. In particular, a group $X^{01}$ of the formula XIXA or XIXB may be introduced at the beginning of, or at any stage during, the synthesis of the penem. In each case, the group $X^{01}$ may suitably be introduced by replacing a hydroxyl group in known manner.

The dehydration or other elimination reaction of the process according to the invention may be carried out at any suitable stage during the preparation of the penem of the general formula I or salt or ester thereof, suitably at an early stage or late stage in the manufacturing process.

Further examples of suitable leaving groups will be apparent to those skilled in the art and include sulphoxide, selenoxide and xanthate groups, which can be eliminated by known methods (see W. Carruthers, 'Some modern methods of organic synthesis', Cambridge Univ. Press 1978 (2nd edition), pages 93-103).

In particular, the dehydration or other elimination reaction may be carried out on a compound of the general formula XIVC:

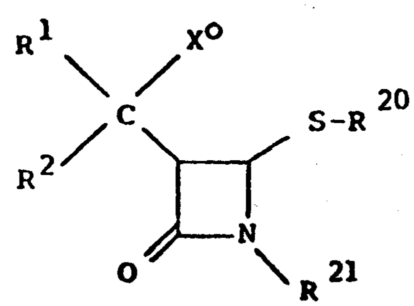

XIVC

in which

$R^1$, $R^2$ and $X^o$ are defined as above,

$R^{20}$ denotes $(C_{1-6})$ alkyl, aryl, aryl$(C_{1-6})$alkyl, $(C_{1-6})$alkylthio, arylthio, hetero-aromatic-thio, acyl (for example, $(C_{1-6})$alkylcarbonyl, especially acetyl), $(C_{2-6})$alkenyl (especially vinyl), or aryl$(C_{2-6})$alkenyl, all of which may optionally be substituted, and

$R^{21}$ denotes hydrogen or an N-protecting group,

to give a compound of the general formula XVC:

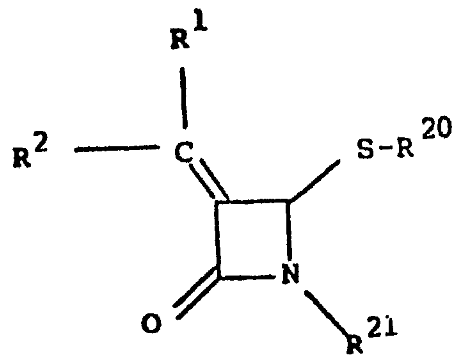

XVC

in which, $R^1$, $R^2$, $R^{20}$ and $R^{21}$ are defined as above,

which may then subsequently be converted to a penem of the general formula I or salt or ester thereof in known manner, suitably by a conventional penem preparation method.

Alternatively, the dehydration or other elimination reaction may be carried out on a compound of the general formula XIVD:

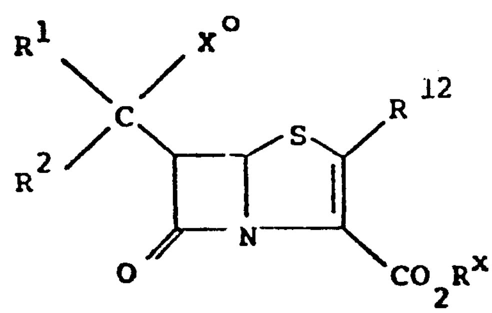

XIVD

in which $R^1$, $R^2$, $R^{12}$, $R^x$ and $X^o$ are defined as above, to give a compound of the general formula XVD:

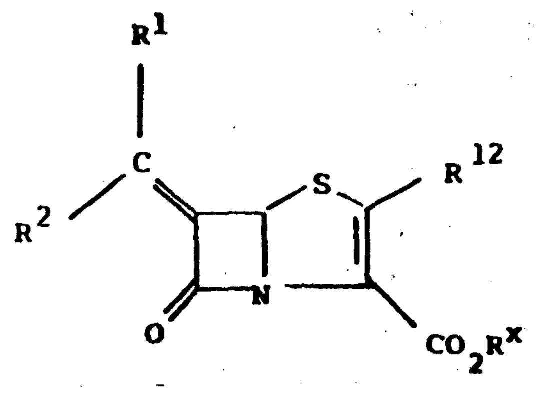

XVD

in which $R^1$, $R^2$, $R^{12}$ and $R^x$ are defined as above, and thereafter, if necessary or desired:

(a) removing any carboxyl-blocking group $R^x$,
and/or
(b) converting the group $R^{12}$ into a group or atom $R^3$,
and/or
(c) converting the product into the free acid or into a pharmaceutically acceptable salt or *in vivo* hydrolysable ester.

The conversion of a compound of the general formula XVC to the desired penem may suitably proceed *via* a compound of the general formula XVD, according to known penem preparation methods.

A compound of the general formula XIVD, especially one in which $X^o$ denotes a leaving group $X^{01}$, may conveniently be prepared from a compound of the general formula XIVC, especially one in which $X^o$ denotes a leaving group $X^{01}$, according to known penem preparation methods.

In a compound of the general formula XIVB or XIVC, $R^{10}$ or $R^{20}$, respectively, may suitably denote a triphenylmethyl group. Examples of suitable N-protecting groups, $R^{11}$ or $R^{21}$, include silyl groups, for example t-butyldimethylsilyl groups.

In the general formula XIVD and XVD, the group $R^{12}$ may be a group convertible into $R^3$ during the penem preparation process. One particular example of such a group, which may conveniently be used in the preparation of a group $R^3$ of the formula $-SR^4$ (in which $R^4$ is defined as above), is the group of the formula XX:

$$-\overset{O}{\overset{\uparrow}{S}}-R^{22} \qquad XX$$

in which $R^{22}$ denotes an organic radical different from the group $R^4$.

A sulphoxide compound of the general formula XIVD or XVD in which $R^{12}$ denotes a group of the formula XX may be reacted with a thiol of the general formula XXI:

$$R^4-SH \qquad XXI$$

in which $R^4$ is defined as above,

or a reactive derivative thereof,

to give a compound of the general formula XIVD or XVD in which $R^{12}$ denotes a group of the formula XXII:

$-S-R^4$ XXII

in which $R^4$ is defined as above.

The reaction of the sulphoxide with the thiol may be carried out as described in European Patent Publication No. EP 0 046 363A.

A sulphoxide compound of the general formula XIVD or XVD in which $R^{12}$ denotes a sulphoxide group of the formula XX above may be prepared by S-oxidation of a compound of the general formula XIVD or XVD, respectively, in which $R^{12}$ denotes a group of the formula $-S-R^{22}$. The S-oxidation may be effected using a mild oxidising agent, for example a perbenzoic acid, hydrogen peroxide, selenium dioxide or sodium metaperiodate. Perbenzoic acids, for example m-chloroperbenzoic acid, are preferred.

The present invention also provides a process for the preparation of a compound of the general formula I in which $R^3$ denotes a group of the formula $-SR^4$ (in which $R^4$ is defined as above), or a pharmaceutically acceptable salt or in vivo hydrolysable ester thereof, which comprises reacting a compound of the general formula XXIII:

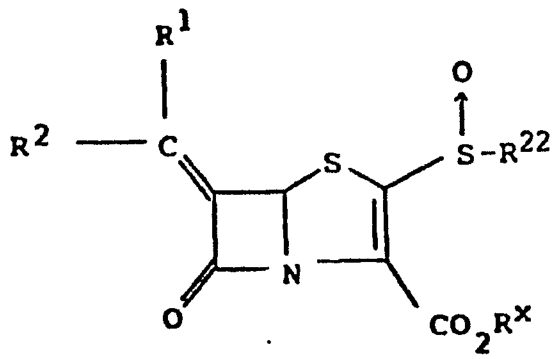

XXIII

in which

$R^1$, $R^2$, $R^{22}$ and $R^x$ are defined as above,

with a thiol of the general formula XXI above, or a reactive derivative thereof;

and thereafter if necessary or desired:

(a) removing any carboxyl-blocking group $R^x$,

and/or

(b) converting the product into the free acid or into a pharmaceutically acceptable salt or <u>in vivo</u> hydrolysable ester.

The compounds of the general formulae XIVC and XVC are novel intermediates and also constitute subjects of the present invention. The compounds of the general formula XIVD, in particular those in which $R^{12}$ denotes $R^3$ or a sulphoxide group of the formula XIX, are also novel intermediates and form a further subject of the present invention. The compounds of the general formula XVD in which $R^{12}$ denotes a sulphoxide group of

the formula XIX are further novel intermediates and constitute a yet further subject of the present invention.

The compounds according to the invention have β-lactamase inhibitory and antibacterial properties, and are useful for the treatment of infections in animals, especially mammals, including humans, in particular in humans and domesticated (including farm) animals. The compounds may be used, for example, for the treatment of infections of, inter alia, the respiratory tract, the urinary tract, and soft tissues, especially in humans.

The compounds may be used for the treatment of infections caused by strains of, for example, Staphylococcus aureus, Klebsiella aerogenes, Escherichia coli, Proteus sp., and Bacteroides fragilis. It is generally advantageous to use a compound according to the invention in admixture or conjunction with a penicillin, cephalosporin or other β-lactam antibiotic and that can often result in a synergistic effect, because of the β-lactamase inhibitory properties of the compounds according to the invention. In such cases, the compound according to the invention and the other β-lactam antibiotic can be administered separately or in the form of a single composition containing both active ingredients as discussed in more detail below.

The compounds according to the invention are suitably provided in substantially pure form, for example at least 50% pure, advantageously at least 75% pure, preferably at least 95% pure, especially at least 98% pure, all percentages being calculated as weight/weight. An impure or less pure form of a

compound according to the invention may, for example, be used in the preparation of a more pure form of the same compound or of a related compound (for example, a corresponding salt, ester or free acid) suitable for pharmaceutical use. Although the purity of any compound used as an intermediate may be less critical than that of a compound used as a final product, for example one used directly for pharmaceutical use (for example in a composition according to the invention as described below), nevertheless such an intermediate compound is advantageously provided in substantially pure form. It is generally advantageous to provide the compounds according to the invention in crystalline form. The free acids and salts according to the invention may be in hydrated or non-hydrated form.

The present invention provides a pharmaceutical composition comprising a compound according to the invention that is to say, a compound of the general formula I or a pharmaceutically acceptable salt or in vivo hydrolysable ester thereof, and a pharmaceutically acceptable carrier.

The present invention also provides a method of treating bacterial infections in animals, especially in humans and in domesticated mammals (including farm mammals), which comprises administering a compound or composition according to the invention to the animal. Such administration may advantageously be effected in conjunction with the prior, simultaneous or subsequent administration of a penicillin, cephalosporin or other β-lactam antibiotic.

The compositions of the invention may be in a form adapted for oral, topical or parenteral use and may be used for the treatment of infection in animals

especially mammals, including humans, in particular in humans and domesticated animals (including farm animals).

The compositions of the invention may, for example, be made up in the form of tablets, capsules, creams, syrups, suspensions, solutions, reconstitutable powders, and sterile forms suitable for injection or infusion. Such compositions may contain conventional pharmaceutically acceptable materials, for example diluents, binders, colours, flavours, preservatives, and disintegrants, in accordance with conventional pharmaceutical practice in manner well understood by those skilled in the art of formulating antibiotics.

It can be particularly advantageous for the compounds according to the invention to be administered to a patient by injection or infusion. That method of administration has the advantage of rapidly resulting in high blood levels of the active ingredient compound being administered. Accordingly, in one preferred form of the composition according to the invention, a compound according to the invention is present in sterile form, including in sterile crystalline form. A further preferred form of the composition according to the invention, is one in which the composition is in injectable or infusable form.

One injectable or infusable form of the composition according to the invention is an injectable or infusable solution, which suitably comprises a solution of a compound according to the invention in a sterile pyrogen-free liquid, for example water or aqueous ethanol.

A further injectable or infusable form of the composition according to the invention is an injectable or infusable suspension, in which case the compound according to the invention is advantageously present in finely particulate form. The suspension may be an aqueous suspension in, for example, sterile water or sterile saline, which may additionally include a suspending agent, for example polyvinylpyrrolidone. Alternatively, the suspension may be an oily suspension in a pharmaceutically acceptable oil suspending agent, for example arachis oil.

A composition according to the invention may be in unit dosage form, for example unit dosage form for oral administration, topical administration, or parenteral administration (including administration by injection or infusion).

A composition according to the invention may comprise a compound according to the invention as the sole active ingredient or therapeutic agent, or it may also comprise one or more additional active ingredients or therapeutic agents, for example a penicillin, cephalosporin or other β-lactam antibiotic, or pro-drug thereof. A composition comprising a compound according to the invention and another active ingredient or therapeutic agent, especially a penicillin, cephalosporin or other β-lactam antibiotic, or pro-drug thereof, can show enhanced effectiveness, and in particular can show a synergistic effect.

Penicillins, cephalosporins and other β-lactam antibiotics suitable for co-administration with the compounds according to the invention - whether by separate administration or by inclusion in the compositions according to the invention - include both

those known to show instability to or to be otherwise susceptible to β-lactamases and also those known to have a degree of resistance to β-lactamases.

Examples of penicillins suitable for co-administration with the compounds according to the invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins. The penicillins may be used in the form of pro-drugs thereof, for example as in vivo hydrolysable esters,for example the acetoxymethyl, pivaloyloxymethyl, α-ethoxycarbonyloxyethyl and phthalidyl esters of ampicillin, benzylpenicillin and amoxycillin; as aldehyde or ketone adducts of penicillins containing a 6α-aminoacetamido side chain (for example hetacillin, metampicillin and analogous derivatives of amoxycillin); and as α-esters of carbenicillin and ticarcillin, for example the phenyl and indanyl α-esters.

Examples of cephalosporins that may be co-administered with the compounds according to the invention include, cefatrizine, cephaloridine, cephalothin, cefazolin, cephalexin, cephacetrile, cephapirin, cephamandole nafate, cephradine, 4-hydroxycephalexin, cephaloglycin, cefoperazone, cefsulodin, ceftazidime, cefuroxime, cefmetazole, cefotaxime, ceftriaxone, and other known cephalosporins, all of which may be used in the form of pro-drugs thereof.

Examples of β-lactam antibiotics other than penicillins and cephalosporins that may be co-administered with the compounds according to the invention include aztreonam,

latamoxef (Moxalactam - Trade Mark), and other known β-lactam antibiotics, all of which may be used in the form of pro-drugs thereof.

In the compositions according to the invention, the compound according to the invention and the penicillin, cephalosporin or other β-lactam antibiotic may be linked by means of an in vivo hydrolysable ester group, in the form of a mutual pro-drug.

Some penicillins and cephalosporins that may be included in the compositions according to the invention may not be suitable for oral administration, in which case the composition will be in a form suitable for parenteral or topical administration.

Particularly suitable penicillins for co-administration with the compounds according to the invention include ampicillin, amoxycillin, carbenicillin, piperacillin, azlocillin, mezlocillin, and ticarcillin. Such penicillins may be used in the form of their pharmaceutically acceptable salts, for example their sodium salts. Alternatively, ampicillin or amoxycillin may be used in the form of fine particles of the zwitterionic form (generally as ampicillin trihydrate or amoxycillin trihydrate) for use in an injectable or infusable suspension, for example, in the manner hereinbefore described in relation to the compounds according to the invention. Amoxycillin, for example in the form of its sodium salt or the trihydrate, is particularly preferred for use in synergistic compositions according to the invention.

Particularly suitable cephalosporins for co-administration with the compounds according to the invention include cephaloridine, cefoperazone and

cefazolin, which may be used in the form of their pharmaceutically acceptable salts, for example their sodium salts.

A compound according to the invention may be administered to the patient in an antibacterially effective amount or, when a compound according to the invention is being used in conjunction with a penicillin, cephalosporin, or other β-lactam antibiotic, it may be used in a synergistically effective amount.

The compounds according to the invention may suitably be administered to the patient at a daily dosage of from 0.1 to 250 mg/kg of body weight. For an adult human (of approximately 70 kg body weight), from 50 to 3000 mg, preferably from 100 to 1000 mg, of a compound according to the invention may be adminstered daily, suitably in from 1 to 6, preferably from 2 to 4, separate doses. Higher or lower dosages may, however, be used in accordance with clinical practice.

When the compositions according to the invention are presented in unit dosage form, each unit dose may suitably comprise from 25 to 1000 mg, preferably from 50 to 500 mg, of a compound according to the invention. Each unit dose may, for example, be 62.5, 100, 125, 150, 200 or 250 mg of a compound according to the invention.

When the compounds according to the invention are co-administered with a penicillin, cephalosporin or other β-lactam antibiotic, the ratio of the amount of the compound according to the invention to the amount of the other β-lactam antibiotic may vary within a wide range. The said ratio may, for example, be from 10:1

to 1:100; more particularly, it may, for example, be from 2:1 to 1:50.

The amount of penicillin or cephalosporin or other β-lactam antibiotic in a synergistic composition according to the invention will normally be approximately similar to the amount in which it is conventionally used per se, for example from about 50 mg, advantageously from about 62.5 mg, to about 3000 mg per unit dose, more usually about 125, 250, 500 or 1000 mg per unit dose.

An example of a particularly suitable composition according to the invention is one comprising from 150 to 1000 mg, preferably from 200 to 700 mg, of amoxycillin or ampicillin or a pro-drug thereof, in admixture or conjunction with from 5 to 500 mg, preferably from 20 to 250 mg, of a compound according to the invention, per unit dose. In such a composition, the amoxycillin may suitably be in the form of its trihydrate or sodium salt; the ampicillin may suitably be in the form of ampicillin trihydrate, ampicillin anhydrate, sodium ampicillin, hetacillin, pivampicillin hydrochloride, bacampicillin hydrochloride or talampicillin hydrochloride; and the compound according to the invention may most suitably be in crystalline form. Such composition may be in a form suitable for oral or parenteral use, except when it comprises an in vivo hydrolysable ester of ampicillin or amoxycillin in which case the composition should not normally be intended for parenteral administration.

A further example of a particularly suitable composition according to the invention is one comprising from 200 to 2000 mg of carbenicillin or

ticarcillin or a pro-drug thereof, in admixture or conjunction with from 5 to 500 mg, preferably from 25 to 250 mg, of a compound according to the invention, per unit dose. In such a composition, the carbenicillin or ticarcillin may most suitably be in the form of its di-sodium salt, and the compound according to the invention may most suitably be in crystaline form. When the composition contains the carbenicillin or ticarcillin in the form of a di-salt, it is most suitably presented in a form suitable for parenteral administration.

No toxicological effects are indicated when the compounds according to the invention are administered within the above-mentioned dosage ranges.

The following examples illustrate the invention. Unless otherwise stated, all temperatures are given in degrees Celsius and all percentages are calculated by weight. The terms 'Biogel' and 'Amberlyst' are Trade Marks.

Preparation 1

i) Methyl (RS) glycerate [adapted from J. Chem. Soc. 1039 (1930)].

Methanolic HCl was prepared by addition of acetyl chloride (17.4 ml) to ice-cooled methanol (250 ml). The cooling bath was removed and calcium RS glycerate dihydrate (25 g) was dissolved in the solution. The solution was refluxed for 3 hours and the solvent evaporated. Methanol was added until the solution was no longer viscous, and this was run through a column of Amberlyst 15 resin (300 g), eluting with methanol. Appropriate fractions were combined and evaporated under reduced pressure. The residue was dissolved in benzene (200 ml) and the solution evaporated: this procedure was repeated to provide crude methyl glycerate (19 g) as an oil.

ii) Methyl (RS) 1,3-dioxolane-4-carboxylate [adapted from Chem. Abs. 63761t (1971)]

Crude methyl (RS) glycerate (19 g), paraformaldehyde (5.6 g), p-toluene sulphonic acid monohydrate (1.6 g) and benzene (50 ml) were mixed and the solvent refluxed under a Dean-Stark for 2 hours. The mixture was cooled, filtered through celite and the filtrate washed with saturated sodium bicarbonate solution, dried over $MgSO_4$ and evaporated under reduced pressure to leave an oil. The oil was distilled (34-8°C, 0.05 mm) to provide methyl RS-1,3-dioxolane-4-carboxylate (7.8 g) as a colourless oil.

Preparation 1(a)

(3S,4R) 1-t-Butyldimethylsilyl-3-[(4R)-1,3-dioxolan-4-ylcarbonyl]-4-tritylthioazetidin-2-one and (3S,4R) 1-t-butyldimethylsilyl-3-[(4S)-1,3-dioxolan-4-ylcarbonyl]-4-tritylthioazetidin-2-one

A solution of (3S,4R) 3-bromo-1-t-butyldimethylsilyl-4-

tritylthioazetidin-2-one (9.7 g) in dry tetrahydrofuran (THF, 60 ml) at -78°C under argon was stirred and treated with a solution of n-butyl lithium (18 mmol) in hexane (10.6 ml). After 15 minutes, methyl (RS)-1,3-dioxolane-4-carboxylate (4.75 g) was added. After a further 15 minutes the reaction was quenched by addition of acetic acid (1.5 ml) in water (50 ml) and diluted with ethyl acetate (200 ml) and brine (100 ml). The mixture was shaken and separated and the organic layer washed with saturated sodium bicarbonate solution, dried over $MgSO_4$ and evaporated under reduced pressure. The residue was chromatographed on silica, eluting with ethyl acetate/hexane (1:9) to separate two products.

The less polar (3S,4R) 1-t-butyldimethylsilyl-3-(1,3-dioxolan-4-ylcarbonyl)-4-tritylthioazetidin-2-one was obtained as a white foam (3.5 g): $\nu_{max.}$ ($CHCl_3$) 1750, 1705 $cm^{-1}$; δ ($CDCl_3$) 0.29 and 0.30 (6H, 2s), 0.93 (9H, s), 3.7 - 3.8 (1H, m), 3.9 - 4.1 (2H, m), 4.36 (1H, d, J 2 Hz), 4.72 (1H, d, J 2 Hz), 4.99 (1H, s), 5.13 (1H, s), 7.1 - 7.5 (15H, m).

The more polar (3S,4R) 1-t-butyldimethylsilyl-3-(1,3-dioxolan-4-ylcarbonyl)-4-tritylthioazetidin-2-one was obtained as a colourless gum (4 g): $\nu_{max.}$ ($CHCl_3$) 1750, 1710 $cm^{-1}$; δ ($CDCl_3$) 0.27 (6H, s), 0.92 (9H, s), 3.8 - 4.0 (2H, m), 4.1 - 4.2 (2H, m), 4.76 (1H, d, J 2 Hz), 4.89 (1H, s), 4.97 (1H, s), 7.1 - 7.5 (15H, m).

Preparation 1(b)

(3S,4R) 1-t-Butyldimethylsilyl-3-[(1,3-dioxolan-4-yl)hydroxymethyl]-4-tritylthioazetidin-2-one

The less polar (3S,4R) 1-t-butyldimethylsilyl-3-(1,3-dioxolan-4-ylcarbonyl)-4-tritylthioazetidin-2-one from

Preparation 1(a) (3.9 g) was dissolved in THF/ethanol (1:1, 40 ml) and ice-cooled. The solution was stirred and treated with sodium borohydride (133 mg). After 10 minutes, the solution was treated with ethyl acetate (150 ml), acetic acid (1 ml) and brine (100 ml). The mixture was shaken and the layers separated. The organic phase was washed with sodium bicarbonate solution (50 ml), dried over $MgSO_4$ and evaporated under reduced pressure. The residue was chromatographed on silica, eluting with ethyl acetate/hexane (1:4 then 1:2) to separate two diastereomers of the title compound. The more polar (3S,4R) 1-t-butyldimethylsilyl-3-[(1,3-dioxolan-4-yl)hydroxymethyl]-4-tritylthioazetidin-2-one (1.44 g, white foam) showed: $\nu_{max.}$ ($CHCl_3$) 3400 (broad), 1730 $cm^{-1}$; δ ($CDCl_3$) 0.12 (3H, s), 0.14 (3H, s), 0.88 (9H, s), 1.81 (1H, d, *J* 8.3 Hz, exchanges with $D_2O$), 3.17 (1H, broad q, collapses to t, *J* 7 Hz on $D_2O$ exchange), 3.48 (1H, dd, *J* 7 and 2 Hz), 3.7 - 3.9 (3H, m), 4.49 (1H, d, *J* 2 Hz), 4.71 (1H, s), 4.83 (1H, s), 7.1 - 7.6 (15H, m).

## Preparation 1(c)

## (3S,4R) 3-[(1,3-Dioxolan-4-yl)hydroxymethyl]-4-tritylthioazetidin-2-one

The more polar (3S,4R) 1-t-butyldimethylsilyl-3-[(1,3-dioxolan-4-yl)hydroxymethyl]-4-tritylthioazetidin-2-one from Preparation 1(b) (1.44 g) was dissolved in methanol (10 ml). The solution was stirred, cooled to -20°C and treated with a solution of anhydrous potassium fluoride (194 mg) in methanol (6 ml). After 1 hour at -20°C to -10°C, the solution was diluted with ethyl acetate (50 ml), washed with brine (2 x 50 ml), dried over $MgSO_4$ and evaporated under reduced pressure. The residue was chromatographed on silica, eluting with ethyl acetate/hexane (2:1), to provide

(3S,4R) 3-[(1,3-dioxolan-4-yl)hydroxymethyl]-4-tritylthioazetidin-2-one as a white foam (1.15 g): $[\alpha]_D^{21}$ -39° (c 1.1, $CHCl_3$); ν max. ($CHCl_3$) 3380, 1760 $cm^{-1}$; δ ($CDCl_3$) 2.69 (1H, d, J 3.5 Hz, exchanges with $D_2O$), 3.37 (1H, broad t, J 2 Hz), 3.8 - 4.1 (4H, m), 4.42 (1H, broad s), 4.56 (1H, d, J 2 Hz) 4.90 (1H, s), 5.11 (1H, s), 7.1 - 7.6 (15H, m).

Preparation 1(d)

(3S,4R) 3-[Acetoxy(1,3-dioxolan-4-yl)methyl]-4-tritylthioazetidin-2-one

The product of Preparation 1(c) (1.15 g) was dissolved in dichloromethane (15 ml) and the solution ice-cooled, stirred and treated with triethylamine (0.43 ml), 4-dimethylaminopyridine (20 mg) and acetic anhydride (0.293 ml). After 10 minutes, the solution was diluted with ethyl acetate (100 ml), washed with 0.5N hydrochloric acid (50 ml), water (50 ml) and saturated sodium bicarbonate solution (50 ml), dried over $MgSO_4$ and evaporated under reduced pressure. The residue was chromatographed on silica, eluting with ethyl acetate/hexane (1:2 then 1:1), to provide the title compound as a white foam (1.25 g): ν max. ($CHCl_3$), 3480, 1765, 1740 (s) $cm^{-1}$; δ ($CDCl_3$) 2.09 (3H, s), 3.37 (1H, ddd, J 5.5, 2.7 and 0.6 Hz), 3.85 (1H, dd, J 8.6 and 5.6 Hz), 3.97 (1H, dd, J 8.6 and 6.8 Hz), 4.1 - 4.3 (2H, m), 4.54 (1H, d, J 2.7Hz), 4.90 (1H, s), 5.16 (1H, s), 5.33 (1H, t, J 5 Hz), 7.1 - 7.6 (15H, m).

Preparation 1(e)

(3S,4R) 3-[Acetoxy(1,3-dioxolan-4-yl)methyl]-1-(1-p-nitrobenzyloxycarbonyl-1-triphenylphosphoranylidene methyl)-4-tritylthioazetidin-2-one

p-Nitrobenzyl glyoxylate monohydrate (700 mg) in benzene (50 ml) was heated to reflux with the provision

for the azeotropic removal of water (Dean and Stark apparatus). A further 100 ml benzene was added at about the same rate as solvent was distilled off, and finally the volume was reduced to 5 ml and cooled.

A solution of the product of Preparation 1(d) (1.25 g) in THF (7 ml) was stirred and treated with the above solution of p-nitrobenzyl glyoxylate and with triethylamine (0.042ml). After 30 minutes the solution was evaporated, toluene was added to the residue and evaporated, and the residue was taken up in dry THF 15 ml). This solution was cooled to -30°C, treated with 2,6-lutidine (0.6 ml) and thionyl chloride (0.28 ml), allowed to reach room temperature and filtered. The filtrate was evaporated, the residue taken up in toluene (20 ml) and filtered through celite. The filtrate was evaporated and the residue dissolved in dry dioxan (7 ml). Triphenylphosphine (4 g) and 2,6-lutidine (0.7 ml) were added and the solution was stirred at 60°C under argon for 11 hours, cooled and evaporated. The residue was chromatographed on silica, eluting with ethyl acetate/hexane (1:2 then 2:1), to provide the title compound as a pale yellow foam (1.8 g): $\nu_{max.}$ ($CHCl_3$) 1745, 1620, 1610, 1520, 1350 $cm^{-1}$.

Preparation 1(f)

(3S,4R) Silver 3-[Acetoxy(1,3-dioxolan-4-yl)methyl]-1-(1-p-nitrobenzyloxycarbonyl-1-triphenylphosphoranylidenemethyl)azetidin-2-one-4-thiolate

A solution of the product of Preparation 1(e) (1.8 g) in dichloromethane (3 ml)/methanol (15 ml) was stirred and treated with pyridine (0.2 ml) and a 0.15M solution of silver nitrate in methanol (11.5 ml). After 45 minutes the precipitate was filtered off and washed

with methanol (5 ml) and ether (20 ml). The filtrate with washings was evaporated to about 10 ml and further precipitate was filtered off and washed with methanol (2 ml) and ether (20 ml). The combined precipitates were dried under vacuum to provide the title compound as an off-white solid (1.33 g).

Preparation 1(g)
(5R,6S) p-Nitrobenzyl 6-[Acetoxy(1,3-dioxolan-4-yl)-methyl]penem-3-carboxylate

A solution of the silver thiolate from Preparation 1(f) (1.33 g) in dichloromethane (25 ml) was ice-cooled, stirred and treated with formic acetic anhydride (1.32 ml), 4-dimethylaminopyridine (0.2 g) and triethylamine hydrochloride (2.28 g). After 30 minutes, the suspension was filtered through celite and the filtrate diluted with ethyl acetate (200 ml), washed with 0.5N hydrochloric acid (100 ml), brine (100 ml) and saturated sodium bicarbonate solution (100 ml), dried over $MgSO_4$ and partially evaporated to about 100 ml. This solution was stored at 5°C for 20 hours, evaporated and the residue was crystallised from dichloromethane/hexane. The mother liquor was evaporated and the residue chromatographed on silica, eluting with ethyl acetate/hexane (1:1). Compound obtained from this was crystallised as above: the crystals were combined to give the title compound as colourless needles (0.5 g): m.p. 161 - 164°C; $[\alpha]_D^{24}$ + 71° (c 0.5, $CHCl_3$): $\nu_{max.}$ (nujol) 1790, 1740, 1720, 1555 $cm^{-1}$; $\lambda_{max.}$ (dioxan) 264 nm (ε 12270), 317 nm (ε 8200); δ ($d_6$-DMSO) 2.07 (3H, s), 3.89 (2H, d, _J_ 5.5 Hz), 4.27 (1H, broad q, _J_ 5 Hz), 4.37 (1H, broad s), 4.75 (1H, s), 4.97 (1H, s), 5.22 (1H, t, _J_ 3.5 Hz), 5.38 (2H, ABq), 5.94 (1H, broad s),

7.68 (2H, d, J 8.5 Hz), 7.94 (1H, s), 8.24 (2H, d, J 8.5 Hz); Found: C, 50.5; H, 3.9; N, 6.1; S, 7.2%; $C_{19}H_{18}N_2O_9S$ requires C, 50.7; H, 4.0; N, 6.2; S, 7.1%).

Example 1(a)

(5R) p-Nitrobenzyl 6-[(1,3-Dioxolan-4-yl)methylene] penem-3-carboxylate

A solution of the product from Preparation 1(g) (478 mg) in dichloromethane (30 ml) was cooled to -40°C, stirred and treated with 1,8-diazabicyclo[5.4.0]undec-7-ene (DBU) (0.24 ml). After 20 minutes 5% aqueous citric acid (50 ml) and ethyl acetate (150 ml) were added and the mixture shaken and separated. The organic layer was washed with brine, dried over $MgSO_4$ and evaporated to about 5 ml. Hexane was added to saturation point and the mixture allowed to crystallise. Filtration provided (5R) p-nitrobenzyl (Z)-6-[(1,3-dioxolan-4-yl)methylene]penem-3-carboxylate (0.25 g) as pale yellow clusters of needles; m.p. 140 - 142°C; $[\alpha]_D^{23}$ -13° (c 0.6, $CHCl_3$); $\nu$ max. (nujol) 1775, 1710, 1550 $cm^{-1}$; $\lambda$ max. (dioxan) 267.5 nm ($\epsilon$ 11060), 294.5 nm ($\epsilon$ 10100); $\delta$ ($d_6$-DMSO) 3,68 (1H, dd, *J* 8 and 6 Hz), 4.10 (1H, t, *J* 8 Hz), 4.7 - 4.9 (2H, m), 4.96 (1H, s), 5.38 (2H, ABq), 6.5 - 6.7 (2H, m), 7.70 (2H, d, *J* 9 Hz), 7.92 (1H, s), 8.25 (2H, d, *J* 9 Hz). Found C, 52.5; H, 3.6; N, 7.1; S, 8.4%. $C_{17}H_{14}N_2O_7S$ requires C, 52.3; H, 3.6; N, 7.2; S, 8.2%.

Example 1(b)

(5R) Sodium (Z)-6-[(1,3-Dioxolan-4-yl)methylene] penem-3-carboxylate

A solution of (5R) p-nitrobenzyl (Z)-6-[(1,3-dioxolan -4-yl)methylene]penem-3-carboxylate (150 mg) in dioxan (10 ml) was treated with water (2 ml) and 5% palladium/

charcoal (225 mg). The mixture was shaken under hydrogen at atmospheric pressure for 1 hour and filtered through celite. The filtrate was treated with 0.1N sodium bicarbonate solution (3.85 ml) and evaporated under reduced pressure to about 2 ml. Water (20 ml) was added and evaporated to about 2 ml. The resulting yellow suspension was added to a column of Biogel P2 and eluted with water. Appropriate fractions were combined and freeze-dried to provide the title compound as a yellow solid (73 mg); $[\alpha]_D^{20}$ $-5.4^o$ (c 0.5, $H_2O$); ν max. (KBr) 1760, 1600, 1550 $cm^{-1}$; λ max. ($H_2O$) 288 nm (ε 5000); δ ($D_2O$) 3.84 (1H, dd, J 8.5 and 5.8 Hz), 4.18 (1H, t, J 8.5 Hz), 4.8 - 5.0 (2H, m), 5.13 (1H, s), 6.47 (1H, dd, J 3.7 and 1 Hz), 6.52 (1H, d, J 1 Hz), 7.08 (1H, s).

Preparation 2(a)

(3S,4R) 1-t-Butyldimethylsilyl-3-[(1,3-dioxolan-4-yl) hydroxymethyl]-4-tritylthioazetidin-2-one

The more polar (3S,4R) 1-t-butyldimethylsilyl-3-(1,3-dioxolan-4-ylcarbonyl)-4-tritylthioazetidin-2-one from Preparation 1(a) (4.0 g) was dissolved in THF/ethanol (1:1, 40 ml) and ice-cooled. The solution was stirred and treated with sodium borohydride (136 mg). After 10 minutes the reaction was worked up and the product chromatographed in an identical manner to that of Preparation 1(b) to separate two diastereomers of the title compound. The less polar compound was obtained as a white foam (2.77 g): ν max. 1735 $cm^{-1}$; δ ($CDCl_3$) 0.01 (3H, s), 0.05 (3H, s), 0.84 (9H, s), 1.6 (1H, broad s), 3.32 (1H, t, 10 Hz), 3.6 - 3.9 (3H, m), 4.04 (1H, broad t, J 6 Hz), 4.28 (1H, d, J 1.5 Hz), 4.81 (1H, s), 4.94 (1H, s), 7.1 - 7.7 (15H, m).

Preparation 2(b)

(3S,4R) 3-[(1,3-Dioxolan-4-yl)hydroxymethyl]-4-tritylthioazetidin-2-one

The less polar product of Preparation 2(a) (2.77 g) was reacted with anhydrous potassium fluoride (302 mg) in methanol (total 25 ml). Reaction, work-up and purification were as in Preparation 1(c) to provide the title compound as a white foam (1.68 g): $[\alpha]_D^{18}$ $-52^o$ (c 1.0, $CHCl_3$); ν max. ($CHCl_3$) 3380, 1765 $cm^{-1}$; δ ($CDCl_3$) 2.46 (1H, broad d, J 6 Hz), 3.24 (1H, dd, J 6 and 2.5 Hz), 3.8 - 3.9 (2H, m), 4.01 (1H, t, J 7.5 Hz), 4.1 - 4.2 (1H, m), 4.27 (1H, broad s), 4.61 (1H, d, J 2.5 Hz), 4.94 (1H, s), 5.14 (1H, s), 7.1 - 7.6 (15H, m).

Preparation 2(c)

(3S,4R) 3-[Acetoxy(1,3-dioxolan-4-yl)methyl]-4-tritylthioazetidin-2-one

The product of Preparation 2(b) (1.68 g) was dissolved in dichloromethane (25 ml), ice-cooled and treated with triethylamine (0.625 ml), 4-dimethylaminopyridine (20 mg) and acetic anhydride (0.425 ml). After 10 minutes the reaction was worked up and chromatographed as described in Preparation 1(d) to provide the title compound as a white foam (1.76 g): ν max. ($CHCl_3$) 3380, 1770, 1735 $cm^{-1}$; δ ($CDCl_3$) 2.18 (3H, s), 3.47 (1H, ddd, J 7.7, 2.7, 1.1 Hz), 3.73 (1H, dd, J 8.7, 5.2 Hz), 3.95 (1H, dd, J 8.7, 7.0 Hz), 4.16 (1H, broad s), 4.31 (1H, ddd, J 7.0, 5.2, 3.3 Hz), 4.49 (1H, d, J 2.7 Hz), 4.96 (1H, s), 5.08 (1H, s), 5.30 (1H, dd, J 7.7, 3.3 Hz), 7.1 - 7.6 (15 H, m).

Preparation 2(d)

(3S,4R) 3-[Acetoxy(1,3-dioxolan-4-yl)methyl]-1-(1-p-nitrobenzyloxycarbonyl-1-triphenylphosphoranylidene methyl)-4-tritylthioazetidin-2-one

p-Nitrobenzyl glyoxylate monohydrate (980 mg) in benzene (50 ml) was heated to reflux with the provision for the azeotropic removal of water (Dean and Stark apparatus). A further 100 ml of benzene was added at about the same rate as distillate was collected, and the resulting clear solution was reduced to about 10 ml and cooled. A solution of the product of Preparation 2(c) (1.76 g) in THF (10 ml) was stirred and treated with the above solution of p-nitrobenzyl glyoxylate and with triethylamine (0.055 ml). After 1 hour, the solvents were evaporated and the residue taken up in toluene and evaporated. The residue was dissolved in THF (20 ml), stirred and cooled to -30°C, treated with 2,6-lutidine (0.87 ml) and thionyl chloride (0.4 ml) and allowed to warm to room temperature. The suspension was filtered and the filtrate evaporated. The residue was taken up in toluene (20 ml), filtered through celite and the filtrate evaporated. The residue was dissolved in dioxan (12 ml), treated with 2,6-lutidine (1 ml) and triphenylphosphine (5.25 g) and stirred under argon at 68°C for 20 hours. The solution was diluted with ethyl acetate (50 ml), washed with 0.5 N hydrochloric acid (50 ml) and brine (50 ml), dried over $MgSO_4$ and evaporated. The residue was chromatographed on silica, eluting with ethyl acetate/hexane (1:2 then 2:1) to give the title compound (2.9 g): $\nu_{max.}$ ($CHCl_3$) 1745, 1615, 1605 $cm^{-1}$.

Preparation 2(e)

(3S,4R) Silver 3-[Acetoxy(1,3-dioxolan-4-yl)methyl]-1-(1-p-nitrobenzyloxycarbonyl-1-triphenylphosphoranylidenemethyl)azetidin-2-one-4-thiolate

The product of Preparation 2(d) (2.9 g) was dissolved in dichloromethane (5 ml)/methanol (20 ml) and treated with pyridine (0.316 ml) and a 0.15 M solution of silver nitrate in methanol (26 ml). Work up was identical to that of Preparation 1(f) to give the title compound as an off-white solid (2.31 g).

Preparation 2(f)

(5R,6S) p-Nitrobenzyl 6-[Acetoxy(1,3-dioxolan-4-yl)methyl]penem-3-carboxylate

A solution of the silver thiolate from Preparation 2(e) (2.31 g) in dichloromethane (30 ml) was ice-cooled, stirred and treated with formic acetic anhydride (2.24 ml), 4-dimethylaminopyridine (0.342 g) and triethylamine hydrochloride (3.86 g). After 30 minutes the reaction was worked up and a solution of the crude product left overnight as described in Preparation 1(g). The solution was evaporated and the residue chromatographed on silica, eluting with ethyl acetate/hexane (1:2) to provide the title compound as a pale yellow foam (1 g); $\nu_{max.}$ ($CHCl_3$) 1795, 1740, 1720, 1560 $cm^{-1}$; $\lambda_{max.}$ (dioxan) 263 nm ($\epsilon$ 12390), 315 nm ($\epsilon$ 7830); $\delta$ ($CDCl_3$) 2.14 (3H, s), 3.8 - 4.0 (2H, m), 4.2 - 4.4 (2H, m), 4.87 (1H, s), 5.08 (1H, m), 5.2 - 5.6 (3H, m), 5.88 (1H, d, *J* 2 Hz), 7.33 (1H, d, *J* 1 Hz), 7.58 (2H, d, *J* 8.6 Hz), 8.23 (2H, d, *J* 8.6 Hz). (Found, $M^+$, 450.072. $C_{19}H_{18}N_2O_9S$ requires 450.073).

Example 2(a)

(5R) p-Nitrobenzyl 6-[(1,3-Dioxolan-4-yl)methylene] penem-3-carboxylate

A solution of the product from Preparation 2(f) (954 mg) in dichloromethane (17 ml) at -40°C was treated with 1,8-diazabicyclo[5.4.0]undec-7-ene (0.456 ml). Workup as in Example 1(a) provided (5R) p-nitrobenzyl (Z)-6-[(1,3-dioxolan-4-yl)methylene]penem-3-carboxylate as pale yellow rectangular plates (0.56 g): m.p. 153-155°C; $[\alpha]_D^{22}$ +86° (c 0.8, DMSO); ν max. (nujol) 1790, 1775, 1705, 1565 $cm^{-1}$; λ max. (dioxan) 268 nm (ε 12100), 295 nm (s) (ε 11000); δ ($d_6$-DMSO) 3.74 (1H, dd, J 8.3, 5.4 Hz), 4.07 (1H, t, J 8 Hz), 4.75 - 4.85 (1H, m), 4.87 (1H, s), 5.07 (1H, s), 5.38 (2H, ABq), 6.58 (1H, dd, J 3.4, 0.9 Hz), 6.62 (1H, s), 7.70 (2H, d, J 9 Hz), 7.90 (1H, s), 8.25 (2H, d, J 9 Hz). (Found C, 52.6; H, 3.5; N, 7.3; S, 7.85%. $C_{17}H_{14}N_2O_7S$ requires C, 52.3; H, 3.6; N, 7.2; S, 8.2%).

Example 2(b)

(5R) Sodium (Z)-6-[(1,3-Dioxolan-4-yl)methylene]penem -3-carboxylate

The product of Example 2(a) (150 mg) was converted into the title compound in exactly the same manner as described in Example 1(b). Thus was produced the title compound as a yellow solid (75 mg): $[\alpha]_D^{22}$ +122°C (c 0.7, $H_2O$); ν max. (KBr) 1770, 1600, 1550 $cm^{-1}$; λ max. ($H_2O$) 289 nm (ε 6020); δ ($D_2O$) 3.85 (1H, dd, J 8.5, 5.2 Hz), 4.16 (1H, t, J 8 Hz), 4.7 - 5.0 (2H, m, partially obscured by HOD), 5.12 (1H, s), 6.4 - 6.5 (2H, m), 7.08 (1H, s).

Preparation 3(a)

(3RS,4SR) 1-t-Butyldimethylsilyl-3-(2,3-epoxybutyryl)-4-tritylthioazetidin-2-one

A solution of n-butyl lithium (2.76M in hexane, 8.7 ml) was added to a solution of diisopropylamine (4 ml) in dry THF (70 ml) at -40°C under a slow stream of nitrogen. After 15 min at -40°C the temperature was dropped to -70°C and the reaction treated with a solution of 1-t-butyldimethylsilyl-4-tritylthioazetidin-2-one (10 g) in dry THF (30 ml). After 10 min the reaction was treated with ethyl 2,3-epoxybutyrate (11 ml) as a single aliquot. After a further six minutes the reaction was quenched with aqueous ammonium chloride. The reaction was diluted with ethyl acetate and washed with brine, dilute acetic acid, and a saturated sodium hydrogen carbonate solution. The organic layer was dried ($MgSO_4$), evaporated, and chromatographed upon silica gel using n-hexane/ethyl acetate (2:1) as eluant. The more polar fraction was isolated and designated as K2, the title azetidinone (2.6 g). $\nu_{max.}$ (film) 1758 and 1712 $cm^{-1}$; $\delta$ ($CDCl_3$) 0.33 (6H, s), 0.95 (9H, s), 1.31 (3H, d, *J* 5Hz), 2.52 - 2.88 (1H, m), 2.9 (1H, d, *J* 2 Hz), 3.75 (1H, d, *J* 2 Hz), 4.79 (1H, d, *J* 2 Hz), 7.14 - 7.68 (15H, m).

Preparation 3(b)

(3RS,4SR) 1-t-Butyldimethylsilyl-3-(2,3-epoxy-1-hydroxybutyl)-4-tritylthioazetidin-2-one

The isomer K2 from Preparation 3(a) was dissolved into dimethoxyethane (40 ml) and cooled to -20°C. Sodium borohydride (0.5 g) was added portionwise. Thin layer chromatography indicated the completion of the reaction

after 1.5 hours. The reaction was diluted with ethyl acetate and washed with citric acid solution before being dried, evaporated and chromatographed upon silica gel using toluene/ethyl acetate (4:1) as eluent. The title alcohol was obtained as a mixture of isomers as a colourless foam (2.58 g), δ ($CDCl_3$) 0.87 (s) and 0.96 (s) (all 9H), 1.21 (d, *J* 5 Hz) and 1.29 (d, *J* 5 Hz) (all 3H), 2.3 - 3.68 (4H, m), 4.3 (d, *J* 2 Hz) and 4.52 (d, *J* 2 Hz) (all 1H), 7.18 - 7.77 (15H, m).

Preparation 3(c)

(3RS,4SR) 3-(2,3-Epoxy-1-hydroxybutyl)-4-tritylthio-azetidin-2-one

The alcohols from Preparation 3(b) were treated as described in Preparation 1(c) to give the title isomeric azetidin-2-ones in essentially pure form without chromatography, 2.04 g. ν max. (film) 3390, 1764 $cm^{-1}$.

Preparation 3(d)

(3RS,4SR) 3-(1-Acetoxy-2,3-epoxybutyl)-4-tritylthio-azetidin-2-one

The alcohols from Preparation 3(c) were treated as described in Preparation 1(d) to give the title azetidinone as a mixture of isomers after chromatographic purification (1.9 g). ν max. (film) 1774 $cm^{-1}$; ($CDCl_3$) 1.34 (d, *J* 2.5 Hz) and 1.42 (d, *J* 2.5 Hz) (all 3H), 2.05 (s) and 2.20 (s) (all 3H), 2.88 - 3.2 (2H, m), 3.4 - 3.72 (2H, m), 4.32 - 5.36 (3H, m), 7.25 - 7.80 (15H, m).

Preparation 3(e)

(3RS,4SR) 3-(1-Acetoxy-2,3-epoxybutyl)-1-(1-p-nitrobenzyloxycarbonyl-1-triphenylphosphoranylidenemethyl)-4-tritylthioazetidin-2-one

The azetidinones from Preparation 3(d) were treated as

described in Preparation 1(e) and thus were converted via the hydroxyesters, ν max. (film) 3420, 1779, 1760 and 1712 $cm^{-1}$ to the title isomeric phosphoranes (3.6 g), ν max. (film) 1751$cm^{-1}$.

Preparation 3(f)

(5RS,6SR) p-Nitrobenzyl 6-(1-Acetoxy-2,3-epoxybutyl)-penem-3-carboxylate

The phosphoranes from Preparation 3(e) were converted to the silver thiolate as described in Preparation 1(f) and then treated as described in Preparation 1(g) to give the title penem as its two acetoxy isomers (0.96 g), ν max. ($CHCl_3$) 1800 and 1720 $cm^{-1}$; ν max. (film) 1790, 1750 and 1723 $cm^{-1}$; δ ($CDCl_3$) 1.32 (3H, d, J 5 Hz), 2.09 (3H, s), 2.86 - 3.25 (2H, m), 4.15 - 4.4 (1H, m), 5 - 5.55 (3H, m), 5.73 and 5.90 (1H, each d, each J 2 Hz), 7.40 (1H, s), 7.65 (2H, d, J 9 Hz), 8.25 (2H, d, J 9 Hz).

Example 3(a)

(5RS) p-Nitrobenzyl 6-(2,3-Epoxybutylidene)penem-3-carboxylate

The penem from Preparation 3(f) was treated as described in Example 1(a) to give the title penem in 66% yield (0.55 g), ν max. ($CHCl_3$) 1787 and 1718 $cm^{-1}$, ν max. (Nujol) 1774 and 1704 $cm^{-1}$; δ ($CDCl_3$) 1.42 (3H, d, J 4.7 Hz), 2.85 - 3.36 (2H, m), 5.22 and 5.53 (2H, ABq, J 14.5 Hz), 6.39 (1H, br s), 6.53 (1H, dd, J 4 Hz and 1 Hz), 7.43 (1H, s), 7.66 (2H, d, J 8.6 Hz), 8.28 (2H, d, J 8.6 Hz).

Example 3(b)

(5RS) Sodium (Z)-6-(2,3-Epoxybutylidene)penem-3-carboxylate

The penem ester from Example 3(a) (0.1 g) was hydrogenated as described in Example 1(b) to give the title salt in 48% yield (0.034 g), as an 8:1 mixture of Z:E isomers, $\lambda_{max.}$ ($H_2O$) 347 nm ($\varepsilon$ 1,300), 289 nm ($\varepsilon$ 5,000) and 226 nm ($\varepsilon$ 12,100); $\nu_{max.}$ (Nujol) 1766 and 1604 $cm^{-1}$; $\delta$ ($D_2O$) 1.36 (3H, d, J 5.1 Hz), 3.19 - 3.29 (1H, m), 3.49 (1H, dd, J 1.94 and 4.75 Hz), 6.42 (1H, s), 6.45 (1H, d, J 4.9 Hz) and 7.08 (1H, s).

Preparation 4(a)

(3RS,4SR) 1-t-Butyldimethylsilyl-3-[(N-benzyloxy-carbonylpyrrolidin-2-yl)carbony]-4-tritylthioazetidin-2-one

A tetrahydrofuran solution of 1-t-butyldimethylsilyl-4-tritylthioazetidin-2-one (10 g) was treated as described in Preparation 3(a). The resulting anion was quenched with racemic methyl-N-benzyloxycarbonylproline (15 g) in tetrahydrofuran (6 ml). Work-up and chromatography upon silica gel using n-hexane/ethyl acetate as eluent (4:1) gave the title azetidinone as an oil (12.55 g), $\nu_{max.}$ (film) 1760, 1724 and 1707$cm^{-1}$; $\delta$ ($CDCl_3$) 0.24 (6H, s), 0.90 (9H, s), 1.5 - 2.2 (4H, m), 3.17 - 3.56 (2H, m), 3.90 - 4.23 (2H, m), 4.44 (1H, br s), 4.93 - 5.17 (2H, br s), 6.93 - 7.53 (19H, m).

Preparation 4(b)

(3RS,4SR) 1-t-Butyldimethylsilyl-3-[(N-benzyloxy-carbonylpyrrolidin-2-yl)hydroxymethyl]-4-tritylthio-azetidin-2-one

The ketone from Preparation 4(a) (9.8 g) was treated as described in Preparation 3(b) to afford, after

purification by silica gel chromatography using toluene/ethyl acetate as eluent (7:1), the title alcohol as separable isomers. The less polar isomer was obtained in 69% yield and the more polar isomer in 27% yield. The less polar isomer showed δ ($CDCl_3$) 0.01 (6H, singlet obscured by T.M.S. internal standard), 0.86 (9H, s), 1.48 - 1.9 (5H, m), 2.9 - 3.75 (5H, m), 4.2 - 4.5 (1H, m), 4.92 - 5.32 (2H, m), 7.0 - 7.8 (19H, m).

Preparation 4(c)

(3RS,4SR) 3-[(N-Benzyloxycarbonylpyrrolidin-2-yl)-hydroxymethyl]-4-tritylthioazetidin-2-one

The less polar isomer from Preparation 4(b) (6 g) was treated as described in Preparation 1(c). The product did not require chromatography and could be crystallized from toluene (4.6 g), M.p. 160 - 162°; $\nu_{max.}$ (Nujol) 3200, 1759 and 1649 $cm^{-1}$; δ ($CDCl_3$) 1.63 - 2.16 (4H, m), 2.99 - 3.59 (3H, m), 3.74 - 4.0 (2H, m), 4.74 (1H, br s), 4.67 (1H, d, J 2 Hz), 5.10 (2H, s), 5.27 - 5.56 (1H, m, exchanges upon addition of $D_2O$), 6.98 - 7.67 (19H, m).

Preparation 4(d)

(3RS,4SR) 3-[Acetoxy(N-benzyloxycarbonylpyrrolidin-2-yl)methyl]-4-tritylthioazetidin-2-one

The azetidinone from Preparation 4(c) (4.6 g) was treated as described in Preparation 1(d). The product did not require chromatographic purification and could be crystallized from acetone (3.83 g), M.p. 214°C; $\nu_{max.}$ (Nujol) 1779, 1743 and 1700 $cm^{-1}$; δ ($CDCl_3$) 1.70 - 2.1 (7H, m), 3.30 - 3.71 (3H, m), 4.16 - 4.72 (3H, m), 5.10 - 5.44 (3H, m), 7.20 - 7.71 (19H, m).

Preparation 4(e)

(3RS,4SR) 3-[Acetoxy(N-benzyloxycarbonylpyrrolidin-2-yl)methyl]-1-(1-p-nitrobenzyloxycarbonyl-1-triphenyl phosphoranylidenemethyl)-4-tritylthioazetidin-2-one

The azetidinone from Preparation 4(d) (3.8 g) was treated as described in Preparation 1(e) to give, after chromatographic purification using n-hexane/ethyl acetate (1:1) as eluent, the title phosphorane (4.52 g), ν max. (Nujol) 1740 and 1690 $cm^{-1}$.

Preparation 4(f)

(5RS,6SR) p-Nitrobenzyl 6-[Acetoxy(N-benzyloxycarbonyl-pyrrolidine-2-yl)methyl]penem-3-carboxylate

The phosphorane from Preparation 4(e) (4.52 g) was treated as described in Preparation 1(f) to give the silver thiolate, ν max. (Nujol) 1740, 1694 and 1600 $cm^{-1}$, which was cyclised as described in Preparation 1(g) to the title penem (1 g), M.p. 143 - 144°C; δ ($CDCl_3$) 1.7 - 2.1 (4H, m), 2.03 (3H, s), 3.25 - 3.75 (2H, m), 4.07 - 4.42 (2H, m), 5.21 (2H, br s), 5.3 - 5.45 (2H, m), 5.45 - 5.62 (1H, m), 5.81 (1H, d, J 2 Hz), 7.26 - 7.76 (8H, m) and 8.28 (2H, d, J 9 Hz).

Example 4(a)

(5RS) p-Nitrobenzyl 6-[(N-Benzyloxycarbonyl-pyrrolidin-2-yl)methylene]penem-3-carboxylate

The penem acetate from Preparation 4(f) (1.0 g) was treated with DBU as described in Example 1(a), to give both the less polar Z isomer (0.53 g), and the more polar E isomer (0.3 g). The Z isomer showed; ν max. (film) 1784 and 1708 br $cm^{-1}$; δ ($CDCl_3$) 1.7 - 2.09 (4H, m), 3.3 - 3.65 (2H, m), 4.28 - 4.7 (1H, m), 5.05 - 5.59 (4H, m), 6.1 - 6.5 (2H, m), 7.3 - 7.41 (6H, br s), 7.55 (2H, d, J 9 Hz), 8.17 (2H, d, J 9 Hz).

Example 4(b)

(5RS) Sodium (Z)-6-[(N-Benzyloxycarbonyl-pyrrolidin-2-yl)methylene]penem-3-carboxylate

The (Z)-ester from Example 4(a) (0.52 g) was hydrogenated as described in Example 1(b) to give the title salt in 60% yield (0.245 g), $\lambda_{max.}$ 288 nm ($\varepsilon$ 8,800) and 207 nm ($\varepsilon$ 30,800); $\nu_{max.}$ (KBr) 1763, 1696 and 1605 $cm^{-1}$; $\delta$ ($D_2O$) (rotomeric), 1.7 - 2.0 and 2.0 - 2.23 (4H, m), 3.3 - 3.5 (2H, m), 4.46 - 4.63 (1H, m), 4.98 - 5.26 (2H, m) 6.15 and 6.28 (1H, each s), 6.36 and 6.44 (1H, each d, J 5.5 and 4.5 Hz respectively), 6.96 and 6.97 (1H, each s), 7.35 and 7.4 (5H, each s).

Preparation 5(a)

(3S,4R)1-t-Butyldimethylsilyl-3-(2,3-epoxy-1-hydroxy butyl)-4-tritylthioazetidin-2-one

(3S,4R) 3-Bromo-1-t-butyldimethylsilyl-4-tritylthio azetidin-2-one (8.5 g) in dry redistilled tetrahydrofuran (35 ml) was cooled to -78°C while under argon; to this solution was added n-butyl lithium in hexane (1.69 M, 9.5 ml) and the resultant deep red solution was allowed to stir for 10 min. The reaction was then quenched with ethyl 2,3-epoxybutyrate dissolved in dry redistilled tetrahydrofuran (5 ml). Having stirred for a further 20 min, an ammonium chloride solution was added before dilution with ethyl acetate. The ethyl acetate was washed with brine (3x) and evaporated. Excess ethyl 2,3-epoxybutyrate was then evaporated at a higher temperature. The residual oil was chromatographed upon silica gel using toluene/ethylacetate (18:1) as eluent. The less polar isomer (designated K1) was readily separable from the more polar isomer, but was not obtained in a pure form

and was thus taken up into dimethoxyethane (30 ml) and treated with sodium borohydride portionwise until all starting material had been consumed (1.5 h). The reaction was then diluted with ethylacetate and washed with citric acid solution before being evaporated and chromatographed upon silica gel using n-hexane/ ethyl acetate (3:1) as eluent. Thus the title epoxide (K1 isomer) was obtained in 16% yield (1.4 g), $\nu_{max.}$ (film) 3425 and 1740cm$^{-1}$; δ ($CDCl_3$) 0.86 (9H, s), 1.16 (3H, d, J 5 Hz), 2.41 - 3.3 (4H, m), 3.46 (1H, dd, J 2 and 7 Hz), 4.41 (1H, d, J 2 Hz) and 7.12-7.7 (15H, m).

Preparation 5(b)

(3S,4R) 3-(2,3-Epoxy-1-hydroxybutyl)-4-tritylthio azetidin-2-one

The azetidinone from Preparation 5(a) (isomer K1, 1.8 g) was reacted as described in Preparation 1(c). Work up and chromatography upon silica gel using n-hexane/ ethyl acetate (1:1) as eluent gave the title azetidin-2-one in 94% yield (1.38 g), $\nu_{max.}$ (film) 3390 and 1764cm$^{-1}$; δ ($CDCl_3$) 1.40 (3H, d, J 5 Hz), 2.63 - 2.95 (2H, m, addition of $D_2O$ causes simplification to 1H, dd, J 2.5 and 3.5 Hz), 2.95 - 3.4 (2H, m), 4.0 - 4.23 (1H, m, simplifies upon addition of $D_2O$), 4.47 - 4.7 (2H, m, simplifies upon addition of $D_2O$ to 1H, d, J 2.5 Hz) and 7. 2 - 7.8 (15H, m).

Preparation 5(c)

(3S,4R) 3-(1-Acetoxy-2,3-epoxybutyl)-4-tritylthio azetidin-2-one

The azetidin-2-one from Preparation 5(b) (1.38 g) was treated as described in Preparation 1(d). The title azetidin-2-one was obtained, without chromatography, by

crystallization from ethyl acetate/n-hexane (1:1) in 82% yield (1.242 g), M.p. 179°; ν max. ($CHCl_3$) 1780 (br) $cm^{-1}$; δ ($CDCl_3$) 1.33 (3H, d, J 5 Hz), 2.06 (3H, s), 2.85 (1H, ddd, J <1, 2 and 4.5 Hz), 3.17 (1H, dd, J 2 and 5 Hz), 3.40 (1H, ddd, J <1, 2.5 and 5.5 Hz), 4.54 (2H, br d, J 2.5 Hz), 5.18 (1H, dd, J 4.5 and 5.5 Hz), 7.2 - 7.77 (15H, m).

Prepartion 5(d)

(3S,4R) 3-(1-Acetoxy-2,3-epoxybutyl)-1-(1-p-nitrobenzyl oxycarbonyl-1-triphenylphosphoranylidenemethyl)-4-tritylthioazetidin-2-one

The acetate from Preparation 5(c) (1.2 g) was treated as described in Preparation 1(e) to give the title phosphorane after purification upon silica gel using n-hexane/ ethyl acetate (1:1) as eluent (1.65 g) (71% yield),
ν max. (film) $1756cm^{-1}$.

Preparation 5(e)

(5R,6S) p-Nitrobenzyl 6-(1-Acetoxy-2,3-epoxybutyl)penem -3-carboxylate

The phosphorane from Preparation 5(d) (1.65 g) was treated as described in Preparation 1(f) to give the silver thiolate (1.34 g). Subsequent treatment as described in Preparation 1(g) gave the title penem in 64% yield (0.47 g), ν max. (film) 1790, 1749 and $1722cm^{-1}$; δ ($CDCl_3$) 1.37 (3H, d, J 5 Hz), 2.15 (3H, s), 2.91 - 3.29 (2H, m), 4.0 - 4.19 (1H, m), 5.30 - 5.60 (3H, m), 5.98 (1H, d, J 2 Hz), 7.43 (1H, s), 7.69 (2H, d, J 9 Hz), 8.31 (2H, d, J 9 Hz).

Example 5(a)

(5R) p-Nitrobenzyl (Z)-6-(2,3-Epoxybutylidene)penem-3-carboxylate

The acetate from Preparation 5(e) (0.47 g) was treated as described in Example 1(a). Purification by chromatography on silica gel using n-hexane/ethyl acetate (1:1) as eluent and crystallization from ethyl acetate/hexane gave the title penem in 50% yield (0.204 g), M.p. 135°; ν max. ($CHCl_3$) 1793 and 1724$cm^{-1}$; δ ($CDCl_3$) 1.39 (3H, d, J 5 Hz), 2.90 - 3.20 (1H, m), 3.2 - 3.38 (1H, m), 5.24 and 5.51 (2H, ABq, J 13 Hz), 6.51 (2H, m), 7.36 (1H, s), 7.64 (2H, d, J 8.5 Hz) and 8.26 (2H, d, J 8.5 Hz).

Example 5(b)

(5R) Sodium (Z)-6-(2,3-Epoxybutylidene)penem-3-carboxylate

The penem from Example 5(a) (0.19 g) was hydrogenated as described in Example 1(b). After chromatography upon Bio-gel P-2, the relevant fractions were combined and freeze-dried. Chromatography over Diaion HP20SS, with water as eluant, then afforded the title salt as a freeze-dried solid (0.014 g), λ max. ($H_2O$) 352 nm (ε 1,350), 285 nm (ε 3,700) and 218 nm (ε 9,150); ν max. (nujol) 1763 and 1604$cm^{-1}$; δ ($D_2O$) 1.37 (3H, d, J 5.3 Hz), 3.27 (1H, dq, J 2.3 and 5 Hz), 3.54 (1H, dd, J 2.3 and 4.7 Hz), 6.44 (1H, d, J 4.7 hz), 6.52 (1H, d, J 0.6 Hz) and 7.7 (1H, s).

Preparation 6

(2S) Ethyl N-(p-methoxybenzyloxycarbonyl)azetidine-2-carboxylate

To an ice cooled suspension of (S)-2-azetidine-carboxylic acid (5g) in dimethylformamide (40ml) was added p-methoxybenzyloxycarbonyl azide (15.4g) and 1,1,3,3-tetramethylguanidine (12.5ml). After addition the cooling bath was removed and the reaction left for 3 days when further aliquots of dimethylformamide (60ml), p-methoxybenzyloxycarbonyl azide (5.1g) and 1,1,3,3-tetramethylguanidine (6.25ml) were added. After a further 3 days the reaction was evaporated and the residue taken up into ethyl acetate and 20% citric acid solution. The organic layer was separated and extracted with potassium carbonate solution which was subsequently acidified with 20% citric acid solution and re-extracted with ethyl acetate. After drying ($MgSO_4$) the ethyl acetate was evaporated leaving a colourless syrup, 8.9g. A dichloromethane (90ml) solution of this syrup was cooled to $0^oC$ and treated sequentially with triethylamine (4.7ml) and triethyloxonium tetrafluoroborate (6.4g). After one hour the reaction was diluted with ethyl acetate and washed with brine (2x), dilute citric acid and finally with sodium bicarbonate solution before being dried ($MgSO_4$) and evaporated to a gum (7.5g), $[\alpha]_D^{20}$ $-69^o$ (c 5.6,$CHCl_3$) $\nu_{max}$ (film) 1737 and 1709 $cm^{-1}$; $\delta(CDCl_3)$ 1.2 (3H, t, J 6.6Hz), 1.9-2.79 (2H, m), 3.76 (3H,s), 3.8-4.85 (5H, m), 5.06 (2H, s), 6.8-7.5 (4H, aromatics.)

Preparation 6(a)

(3S,4R)1-t-Butyldimethylsilyl-3-[(2S)-N-p-methoxybenzyl-oxycarbonylazetidin-2-ylcarbonyl]-4-tritylthioazetidin-2-one

A tetrahydrofuran (40ml) solution of (3S 4R) 3-bromo-1-t-butyldimethylsilyl-4-tritylthioazetidin-2-one (8g) was treated with n-butyl lithium, as in Preparation 1(a). The resulting anion was quenched after five minutes with (2S) ethyl N-(p-methoxybenzyloxycarbonyl)azetidine-2-carboxylate (6.0gm). Saturated ammonium chloride solution was added after a further twelve minutes prior to dilution with ethyl acetate. After a brine wash and evaporation of solvent, the residue was chromatographed using n-hexane/ethyl acetate (3:1) as eluent to give the title ketone (3.943g). $[\alpha]_D^{20}$-18.5°(c 4.0,$CHCl_3$); $\nu_{max}$ (KBr) 1753 and 1705 (br) $cm^{-1}$.

Preparation 6(b)

(3S,4R)3-(Hydroxy[(2S)-N-p-methoxybenzyloxy-carbonylazetidin-2-yl]methyl)-4-tritylthioazetidin-2-one

The ketone (3.8g) from Preparation 6(a) was treated as for Preparation 1(b) with the exception that the crude product was taken up into methanol (30ml) and treated as for Preparation 1(c). The product was chromatographed upon silica gel using n-hexane/ethyl acetate (1:1) as eluent to give the title alcohol (2.42g), $\nu_{max}$ (film) 3530, 3360, 1747 and 1710 $cm^{-1}$.

Preparation 6(c)

(3S,4R)3-(Acetoxy[(2S)-N-p-methoxybenzyloxycarbonyl-azetidin-2-yl]methyl)-4-tritylthioazetidin-2-one

A dichloromethane (20ml) solution of the alcohol from Preparation 6(b)(2.23g) was treated as for Preparation 1(d). Silica gel chromatography using toluene/ethyl acetate (4:1) as eluent afforded the title acetate (1.733g), $\nu_{max}$ (film) 3380, 3260, 1774, 1740 and 1704cm$^{-1}$.

Preparation 6(d)

(3S,4R) 3-(Acetoxy[(2S)-N-p-methoxybenzyloxycarbonyl-azetidin-2-yl]methyl)-1-(1-p-methoxybenzyloxycarbonyl-1-triphenylphosphoranylidenemethyl)-4-tritylthio-azetidin-2-one

The acetate from Preparation 6(c)(1.733g) was reacted with p-methoxybenzyl glyoxalate (0.64g) according to the procedure described in Preparation 1(e). Treatment of the resulting hydroxyester with thionyl chloride, followed by triphenylphosphine afforded the title phosphorane (1.623g) after silica gel column chromatography, using n-hexane/ethyl acetate (1:1) as eluant, $\nu_{max}$ (film) 1749, 1700 and 1609cm$^{-1}$.

Preparation 6(e)

(3S,4R) Silver 3-(Acetoxy[(2S)-N-p-methoxybenzyloxy-carbonylazetidin-2-yl]methyl)-1-(1-p-methoxybenzyloxy-carbonyl-1-triphenylphosphoranylidenemethyl)azetidin-2-one-4-thiolate

The phosphorane (1.623g) from Preparation 6(d) was treated as for Preparation 1(f) to give the title thiolate, (0.866g).

Preparation 6(f)

(5R,6S) p-Methoxybenzyl 6-(Acetoxy[(2S)-N-p-methoxybenzyloxycarbonylazetidin-2-yl]methyl)penem -3-carboxylate

The silver thiolate (0.866g) from Preparation 6(e) was treated as for Preparation 1(g). After drying ($MgSO_4$), the ethyl acetate solution was warmed to 40°c for 0.5 hours. Subsequent evaporation and silica gel chromatography, using n-hexane/ethyl acetate (1:1) as eluent gave the title penem (0.548g), $[\alpha]_D$ -3.8(c 1.74, $CHCl_3$); $\nu_{max}$ (film) 1796, 1745, 1711 and 1705(sh)$cm^{-1}$; $\delta(CDCl_3)$ 1.56-2.66 (5H, m), 3.5-4.02 (8H, m), 4.02-4.25 (1H, m), 4.25-4.66 (1H, m), 4.99 and 5.10 each (2H, broad s), 5.31-5.51 (1H, m), 5.66 (1H broad d) and 6.60-7.40 (9H, m).

Example 6(a)

(5R) p-Methoxybenzyl (Z)-6-([(2S)-N-p-methoxybenzyloxycarbonylazetidin-2-yl]methylene)penem-3-carboxylate

The acetate (0.473g) from Preparation 6(f) was treated as for Example 1(a), though the crude product was chromatographed on silica gel using n-hexane/ethyl acetate (1:1) as eluent. The title penem was thus obtained as a foam (0.418g), $\nu_{max}$(film) 1780 and 1705$cm^{-1}$.

Example 6(b)

(5R) (Z)-6-([(2S)-Azetidin-2-yl]methylene)penem-3-carboxylic acid

A solution of the penem from Example 6(a)(0.0647g) in anisole (0.22ml) was cooled under argon to -50°C. A suspension of aluminium trichloride (0.08g) in dichloromethane (0.8ml) was added and after twenty minutes phosphate buffer (0.01M, 1.6ml) quenched the

reaction. The reaction was diluted with deionized water and washed with ethyl acetate. The aqueous layer was concentrated and passed down a Diaion HP20SS column, eluting with deionized water. Fractions exhibiting u.v. absorptions at 287.7 and 212.5nm were combined and freeze-dried to give the title penem (0.0024g), $\nu_{max}$ (Nujol Mull) 1765 and 1590cm$^{-1}$; $\delta(D_2O)$ 2.5-2.86 (2H, m), 3.87-4.25 (2H, m), 4.6-5.1 (signals obscured by HOD), 6.43 (1H, s), 6.71 (1H, d, *J* 6.3 Hz) and 7.11 (1H, s).

Preparation 7

Methyl 3,4-Dihydro-2H-pyran-2-carboxylate

A solution of sodium 3,4-dihydro-2H-pyran-2-carboxylate (12g) in dimethylformamide (100ml) containing iodomethane (4.1 ml) was stirred for 16 hours. The reaction was then diluted with ethyl acetate and washed thoroughly with saturated brine (5x) before careful evaporation of solvent. The crude product was purified by distillation at water-pump pressure (5g) b.p. 78-80°C, $\delta(CDCl_3)$ 1.81-2.36 (4H, m), 3.84 (3H, s), 4.37-4.99 (2H, m),6.46 (1H, br d, *J* 6Hz).

Preparation 7(a)

(3RS, 4SR) 1-t-Butyldimethylsilyl-3-[(3,4-dihydro-2H-pyran-2-yl)carbonyl]-4-tritylthioazetidin-2-one

The anion of 1-t-butyldimethylsilyl-4-tritylthioazetidin-2-one (10g), formed according to Preparation 3(a), was quenched by the addition of the methyl 3,4-dihydro-2H-pyran-2-carboxylate (3.9g) in tetrahydrofuran (6ml). The crude product could be isolated by crystallization from n-hexane after work up according to the procedure in Preparation 3(a), (11.3g), m.p. 121-123°C; $\delta(CDCl_3)$ 0.90 (9H, s), 1.65-2.20 (4H, m), 3.80-4.15 (1H, m), 4.3-4.9 (3H, m) 6.41 (1H, br d, *J* 6.6 Hz) and 7.15-7.7 (15H, m).

Preparation 7(b)

(3RS, 4SR) 1-t-Butyldimethylsilyl-3-[(3,4-dihydro-2H-pyran-2-yl)hydroxymethyl]-4-tritylthioazetidin-2-one

The ketone (10g) from Preparation 7(a) was dissolved in dimethoxyethane (50ml) and treated as for Preparation 1(b). The crude products were chromatographed on silica gel using toluene/ethyl acetate (10:1) as eluent to give the most polar isomer of the title alcohol (3g), $\nu_{max}$ (film) 3440, 1744 and 1648cm$^{-1}$.

Preparation 7(c)

(3RS,4SR) 3-[(3,4-Dihydro-2H-pyran-2-yl)hydroxymethyl]-4-tritylthioazetidin-2-one

A solution of the alcohol from Preparation 7(b)(3g) in methanol (30ml) and dimethoxyethane (15ml) was treated as for Preparation 1(c). The crude product was purified by silica gel chromatography using toluene/ethyl acetate (7:1) as eluent to give the title azetidinone as a foam (2.4g), $\nu_{max}$ (nujol) 3390, 2520, 1750 and 1643cm$^{-1}$.

Preparation 7(d)

(3RS,4SR) 3-[Acetoxy(3,4-dihydro-2H-pyran-2-yl)methyl]-4-tritylthioazetidin-2-one

The azetidinone (2.4g) from Preparation 7(c) was treated as for Preparation 1(d) to give the title acetate (2g), $\nu_{max}$ (nujol) 1770, 1747 and 1651cm$^{-1}$; $\delta$($CDCl_3$) 1.5-2.3 (5H, m), 3.4-3.62 (1H, m), 3.8-4.41 (2H, m), 4.59 (1H, d, *J* 1.5Hz), 4.66-4.96 (1H, m), 5.37(1H, t, *J* 2.6Hz), 6.51 (1H, d, *J* 3.5 Hz), 7.16-7.81 (15H, m).

Preparation 7(e)

(3RS,4SR) 3-[Acetoxy(3,4-dihydro-2H-pyran-2-yl)methyl]-1-(1-p-nitrobenzyloxycarbonyl-1-triphenylphosphoranylidenemethyl)-4-tritylthioazetidin-2-one

The azetidinone from Preparation 7(d)(2g) was treated as for Preparation 1(e) to give the title phosphorane (3.6g), $\nu_{max}$ (film) 1745, 1644, 1620 and 1603cm$^{-1}$.

Preparation 7(f)

(5RS,6SR) p-Nitrobenzyl 6-[Acetoxy(3,4-dihydro-2H-pyran-2-yl)methyl]penem-3-carboxylate

The phosphorane from Preparation 7(e)(3.6g) was treated as for Preparation 1(f). The resulting silver salt was not isolated. Instead, the solvent was removed in vacuo and the residue was taken up into dichloromethane and treated as for Preparation 1(g). After work up, the solution of reaction products was heated to 40°C for fifteen minutes. Evaporation of solvent and silica gel purification using n-hexane/ethyl acetate (2:1) as eluent gave the title penem (0.4g), $\nu_{max}$ (nujol) 1796, 1742, 1713, 1647 and 1605cm$^{-1}$; $\delta$($CDCl_3$) 1.5-2.34 (7H, m), 3.9-4.32 (2H, m), 5.15-5.57 (3H, m), 5.99 (1H, d, J 1Hz), 6.37 (1H broad, d, J 3.5Hz), 7.2-8.51 (6H, m).

Example 7(a)

(5RS) p-Nitrobenzyl 6-[(3,4-Dihydro-2H-pyran-2-yl)methylene]penem-3-carboxylate

The penem acetate from Preparation 7(f) (0.4g) was treated as for Example 1(a) but with a reaction time of 6 minutes. Purification of the products by silica gel chromatography using toluene/ethyl acetate (7:1) as

eluent gave the title penem (0.146g), m.p. 132-133°C (diethyl ether); $\nu_{max}$ 1790, 1718, 1650 and 1609cm$^{-1}$; δ($CDCl_3$) 1.68-2.24 (4H, m), 4.6-4.68 (1H, m), 4.74-4.85 (1H, m), 5.28 and 5.44 (2H, ABq, J 13.6Hz), 6.33-6.6 (2H, m), 6.48 (1H, s), 7.36 (1H, s), 7.61 (2H, d, J 8.7Hz), 8.23 (2H, d, J 8.7Hz).

Example 7(b)

(5RS) Sodium (Z)-6-[(3,4-Dihydro-2H-pyran-2-yl) methylene]penem-3-carboxylate

The penem from Example 7(a) (0.146g) was treated as for Example 1(b) to give the title salt as a freeze-dried solid (0.044g), $\nu_{max}$ (nujol) 1759, 1645 and 1599cm$^{-1}$; δ($D_2O$) 1.65-2.2 (4H, m), 4.7-4.85 (1H, m, partly obscured by HOD), 4.85-4.93 (1H, m), 6.42 (1H, d, J 6.1Hz), 6.45-6.54 (2H, m) and 7.06 (1H, s).

Preparation 8

Methyl (S) 2-Hydroxy-N-(methyloxy)succinamate

(L)-1-Methyl hydrogen malate (9.26g)[see Miller et al, J.Org.Chem.,(1982),47,4928] and methoxylamine hydrochloride (7.8g) were dissolved in water (220ml) and brought to pH 4.5 by the dropwise addition of 1N aqueous sodium hydroxide. A solution of 1-(3-dimethylaminopropyl)-3-ethylcarbodiimide hydrochloride (25g) in water (80ml) was added. The pH of the solution was maintained at 4.5 by addition of 1N hydrochloric acid for 1.5 hours. The solvent was evaporated at reduced pressure and n-propane (300ml) was added to the residue and evaporated at reduced pressure. The residue was chromatographed over silica, eluting with ethyl acetate/ethanol (4:1), to provide the title compound as a colourless oil (10.4g), $\nu_{max}$($CHCl_3$) 3400, 3360, 1740 and 1690cm$^{-1}$; δ($D_2O$:HOD=4.60δ) 2.55 (2H, d, J 6Hz), 3.64 (3H, s) and 3.70 (3H, s).

Methyl (R) 1-Methyloxyazetidin-2-one-4-carboxylate

Methyl (S) 2-hydroxy-N-(methyloxy)succinamate (10.2g) and triphenylphosphine (15.1g) were dissolved in dry tetrahydrofuran (300ml) and the solution cooled in an ice bath. Diethylazodicarboxylate (9.4ml) was added and the solution was stored for 18 hours in a refrigerator and evaporated under reduced pressure. The residue was chromatographed on silica, eluting with ethyl acetate/hexane (1:2) to provide the title compound contaminated with diethyl hydrazodicarboxylate. This mixture was taken up in carbon tetrachloride (200ml), left to stand for 2 hours and the crystalline diethyl hydrazodicarboxylate filtered off. The filtrate was evaporated and the residue dried under vacuum to provide the title compound as a gum which crystallised on standing (7.8g); $[\alpha]_D^{20}$ + 135°(c 9.5, $CHCl_3$); $\nu_{max}$ 1780, 1740cm$^{-1}$; $\delta(CDCl_3)$ 2.5-3.2 (2H, m), 3.82 (3H, s), 3.87 (3H, s) 4.40 (1H, dd, *J* 5.5 and 3.5Hz).

Preparation 8(a)

(3S,4R) 1-t-Butyldimethylsilyl-3-[(4R)-1-methyloxy-2-oxoazetidin-4-ylcarbonyl]-4-tritylthioazetidin-2-one

The anion formed from (3S,4R) 3-bromo-1-t-butyldimethylsilyl-4-tritylthioazetidin-2-one (6.3g) by the method employed in Preparation 1(a) was quenched with a solution of methyl (R) 1-methyloxyazetidin-2-one-4-carboxylate (2.2g) in tetrahydrofuran (8ml). After a reaction time of 8 minutes and work up as for Preparation 3(a), the products were purified by silica gel chromatography using n-hexane/ethyl acetate (3:1) as eluent, thus giving the title ketone (3.2g), $\nu_{max}$ (KBr) 1786, 1754 and

$1713cm^{-1}$; $\delta(CDCl_3)$ 0.91 (9H, s), 2.35 and 2.74 (2H, dABq, J 3 and 6Hz), 3.73 (3H, s), 3.98 (1H, d, J 2Hz), 4.32 (1H, dd, J 3 and 6Hz), 4.81 (1H, d, J 2Hz) and 7.14-7.62 (15H, m).

Preparation 8(b)
(3S,4R) 1-t-Butyldimethylsilyl-3-(hydroxy[(4R)-1-methyloxy-2-oxoazetidin-4-yl]methyl)-4-tritylthioazetidin-2-one

The ketone from Preparation 8(a) (3.2g) was dissolved in dimethyoxyethane (30ml) and reduced as for Preparation 1(b). Purification by silica gel chromatography using n-hexane/ethyl acetate (2:1) as eluent afforded the title alcohol as a 1:1 mixture of isomers (2.35g), $\nu_{max}$ $(CH_2Cl_2)$ 1774 and $1741cm^{-1}$; $\delta(CDCl_3)$ 0.067, 0.11, 0.30 and 0.34 each (3H, s), 0.87 and 0.96 each (9H, s), 1.66 (1H, broad s, isomer B), 1.94 and 2.66 (2H, ABX, J 2.3, 5.3 and 13.7Hz, isomer A), 2.05 (1H, m, isomer A), 2.44 (2H, m, isomer B), 2.54 (1H, oct, J 2.5, 4.1 and 9.2 Hz, isomer A), 2.97 (1H, m, isomer A), 3.37 (1H, dd, J 9.99 and 1.4Hz, isomer B), 3.736 and 3.738 each (3H, s, isomers A and B), 3.81 (1H, oct, J 1.6, 6.8 and 9.8 Hz, isomer B), 3.94-4.2 (1H, m, isomer B), 4.13 (1H, oct, J 2.3, 5.3 and 9.2Hz, isomer A), 4.22 (1H, d, J 1.4Hz, isomer B), 4.47 (1H, d, J 1.9Hz, isomer A) and 7.17-7.59 (30H, m, isomers A and B).

Preparation 8(c)
(3S,4R) 3-(Hydroxy[(4R)-1-methyloxy-2-oxoazetidin-4-yl]methyl)-4-tritylthioazetidin-2-one

The alcohols from Preparation 8(b)(2.1g) were treated as for Preparation 1(c) to give the title alcohol as a 1:1 mixture of isomers (1.4g), $\nu_{max}$ (film) 3375 and 1762 (br) $cm^{-1}$.

(Preparation 8(d)

(3S,4R) 3-(Acetoxy[(4R)-1-methyloxy-2-oxoazetidin-4-yl]methyl)-4-tritylthioazetidin-2-one

The alcohols from Preparation 8(c) were treated as for Preparation 1(d) to give the two acetates. Isomer I (0.606g) exhibited $\nu_{max}$ (film) 3380, 3260 and 1778cm$^{-1}$; $\delta(CDCl_3)$ 2.23 (3H, s), 2.72-2.8 (2H, m), 3.19 (1H, broad d, *J* 9.4Hz), 3.73 (3H, s), 4.17-4.24 (1H, m), 4.25-4.34 (1H, broad s), 4.44 (1H, broad s), 5.60 (1H broad d, *J* 9.6Hz), 7.21-7.6 (15H, m). Isomer II (0.52g) exhibited $\nu_{max}$ (film) 3395, 3275, 1780(br) and 1750(sh) cm$^{-1}$; $\delta(CDCl_3)$ 2.04 (3H, s), 2.62 and 2.82 (2H, ABX, *J* 2.7, 5.5 and 14 Hz), 3.37 (1H, oct, *J* <1, 3 and 4.5Hz), 3.77 (3H, s), 4.20 (1H, oct, *J* 2.7, 5.6 and 7Hz), 4.26 (1H, d, *J* 3Hz), 4.52 (1H, broad s), 5.38 (1H, dd, *J* 4.5 and 7Hz) and 7.2-7.51 (15H, m).

Preparation 8(e)

(3S,4R) 3-(Acetoxy[(4R)-1-methyloxy-2-oxoazetidin-4-yl]methyl)-1-(1-p-nitrobenzyloxycarbonyl-1-triphenylphosphoranylidenemethyl)-4-tritylthioazetidin-2-one

The combined acetates (1.126g) from Preparation 8(d) were treated as for Preparation 1(e) to give the title phosphorane after silica gel chromatography using n-hexane/ethyl acetate (1:1) as eluent (1.833g), $\nu_{max}$ (film) 1779, 1752 and 1628cm$^{-1}$.

Preparation 8(f)

(5R,6S) p-Nitrobenzyl 6-(Acetoxy[(4R)-1-methyloxy-2-oxoazetidin-4-yl]methyl)penem-3-carboxylate

The phosphoranes from Preparation 8(e)(1.833g) were treated as for Preparations 1(f) and 1(g) to give the title penem acetates after silica gel purification using n-hexane/ethyl acetate (1:2) as eluent (0.524g), $\nu_{max}$ (film) 1782(br), 1744 and 1720cm$^{-1}$; $\delta(CDCl_3)$ 2.15 (3H, s, isomer A), 2.18 (3H, s, isomer B), 2.74 and 2.92 (2H, ABX, J 2.7, 5.6 and 14.1Hz, isomer B), 2.76 and 2.87 (2H, ABX, J 2.8, 5.3 and 13.8Hz, isomer A), 3.77 (3H, s, isomer A), 3.81 (3H, s, isomer B), 3.97 (1H, oct, J 1, 1.7 and 7.9Hz, isomer A), 4.11 (1H, oct, J 1.1, 1.9 and 3.7Hz, isomer B), 4.21 (1H, oct, J 2.6, 2.8 and 5.3Hz, isomer A), 4.27 (1H, oct, J 2.7, 5.6 and 7Hz, isomer B), 5.28 and 5.43 (4H, superimposed 2 x ABq, J 13.4Hz, isomers A and B), 5.52 (1H, dd, J 3.7 and 7Hz, isomer B), 5.63 (1H, dd, J 2.6 and 7.9Hz, isomer A), 5.67 (1H, d, J 1.9Hz, isomer B), 5.86 (1H, d, J 1.7Hz, isomer A), 7.32 (1H, d, J 1.1Hz, isomer B), 7.34 (1H, d, J 1Hz, isomer A), 7.57 (4H, 2xd, J 8.7Hz, isomers A and B), 8.24 (4H, 2xd, J 8.7Hz isomers A and B).

Example 8(a)

(5R) p-Nitrobenzyl 6-([(4R)-1-Methyloxy-2-oxoazetidin-4-yl]methylene)penem-3-carboxylate

The mixed acetates from Preparation 8(f) (0.5g) were treated as for Example 1(a). Purification by silica gel chromatography using n-hexane/ethyl acetate (1:2) as eluent afforded two isomers. The first eluted isomer was (5R) p-nitrobenzyl (E)-6-([(4R)-1-methyloxy-2-oxoazetidin-4-yl]methylene)-penem-3-carboxylate (0.19g), $[\alpha]_D^{20}$-214° (c 2.6, $CHCl_3$); $\nu_{max}$ ($CHCl_3$) 1780 and 1720cm$^{-1}$; $\delta$($CDCl_3$) 2.59 and 3.10 (2H, ABX, J <2, 5.2 and 14 Hz), 3.81 (3H, s), 5.14-5.57 (3H, m), 5.91 (1H, broad d, J 9.6Hz), 6.36 (1H, broad s), 7.43 (1H, s), 7.61 (2H, d, J 8.4Hz) and 8.24 (2H, d, J 8.4Hz). The second eluted isomer was (5R) p-nitrobenzyl (Z)-6-([(4R)-1-methyloxy-2-oxo-azetidin-4-yl]methylene)penem-3-carboxylate (0.222g), $[\alpha]_D^{20}$ + 6.6°(c 2.7, $CHCl_3$); $\nu_{max}$ ($CHCl_3$) 1781 and 1720cm$^{-1}$; $\delta$($CDCl_3$) 2.68 and 3.09 (2H, ABX, J 2.5, 5.8 and 13.8Hz), 3.85 (3H, s), 4.59 (1H, oct, J 2.5, 5.8 and 5.9Hz), 5.29 and 5.44 (2H, ABq, J 13.6Hz), 6.4-6.52 (2H, m), 7.40 (1H, s), 7.61 (2H, d, J 8.7Hz) and 8.23 (2H, d, J 8.7Hz).

Example 8(b)

(5R) Sodium (Z)-6-([(4R)-1-Methyloxy-2-oxoazetidin-4-yl]methylene)penem-3-carboxylate

The second eluted, (Z)-isomer from Example 8(a) (0.222g) was treated as for Example 1(b) to give the title salt as a freeze-dried solid (0.093g), $\nu_{max}$ (nujol) 1764 and 1600cm$^{-1}$; $\delta$($D_2O$) 2.73 and 3.14 (2H, ABX, J 2.1, 5.3 and 14Hz), 3.80 (3H, s), 4.87 (1H, oct, J 2.1, 5.3 and 6.4Hz), 6.48 (1H, d, J 0.9Hz), 6.53 (1H, dd, J 0.9 and 6.4Hz), and 7.08 (1H, s).

Example 8(c)

(5R) Sodium (E)-6-([(4R)-1-Methyloxy-2-oxoazetidin-4-yl]methylene)penem-3-carboxylate

The first eluted, (E)-isomer from Example 8(a) (0.19g) was treated as for Example (1b) to give the title salt as a freeze-dried solid (0.055g), $\nu_{max}$ (nujol) 1760,

1740 and 1600$cm^{-1}$; $\delta(D_2O)$ 2.69 and 3.12 (2H, ABX, J 2.0, 5.1 and 14.1Hz), 3.78 (3H, s), 5.24 (1H, oct, J 2.0, 5.1 and 9.8Hz), 6.17 (1H, d, J 9.8Hz), 6.35 (1H, s), and 7.09 (1H, s).

Preparation 9(a)

(3S,4R) 1-t-Butyldimethylsilyl-3-[(5R)-3-methyl-2-isoxazolin-5-ylcarbonyl]-4-tritylthioazetidin-2-one and (3S,4R) 1-t-Butyldimethylsilyl-3-[(5S)-3-methyl-2-isoxazolin-5-ylcarbonyl]-4-tritylthioazetidin-2-one

A solution of (3S,4R) 3-bromo-1-t-butyldimethylsilyl-4-tritylthioazetidin-2-one in dry tetrahydrofuran was treated with n-butyl lithium as for Preparation 1(a). The resulting anion was quenched with methyl (R,S) 3-methyl-2-isoxazoline-5-carboxylate. After a reaction time of 20 minutes the reaction was further quenched with saturated ammonium chloride solution and worked up as for Preparation 1(a). Purification by chromatography on silica eluting with ethyl acetate/n-hexane (1:3) gave two products. The less polar isomer (designated isomer K1) was isolated as a crystalline solid from diethylether/n-hexane (33% yield), M.p. 115-116°C; $\nu_{max}$ ($CHCl_3$) 1752 and 1711$cm^{-1}$. The more polar isomer (designated isomer K2) was isolated as a gum (36% yield), $\nu_{max}$ (film) 1752 and 1712$cm^{-1}$.

Preparation 9(b)

(3S,4R) 3-[Hydroxy(3-methyl-2-isoxalin-5-yl)methyl]-4-tritylthioazetidin-2-one

The less polar isomer (isomer K1) from Preparation 9(a) was dissolved in dimethoxyethane and ice-cooled.

Sodium borohydride addition and work up as for Preparation 1(b) gave a crude product, this was passed through a short plug of silica with toluene/ethyl acetate (2:1) as eluent. The isolated gum was redissolved in methanol and treated with potassium fluoride as for Preparation 1(c). After 15 minutes at ambient temperature the reaction was diluted with ethyl acetate and washed with brine (3 x) and with saturated sodium hydrogen carbonate. After evaporation the residue was chromatographed on silica, eluting with n-hexane/ethyl acetate (1:2), to provide the title alcohol as a foam (36% yield), $\nu_{max}$(film) 3390 and 1760cm$^{-1}$.

Preparation 9(c)
(3S,4R) 3-[Acetoxy(3-methyl-2-isoxalin-5-yl)methyl] -4-tritylthioazetidin-2-one

The alcohol from Preparation 9(b) was dissolved in dichloromethane, cooled to 0°C and treated with acetic anhydride as for Preparation 1(d). After 20 minutes the reaction was diluted with ethyl acetate and washed with brine (2 x) followed by dilute citric acid solution. Evaporation and chromatography on silica, eluting with n-hexane/ethyl acetate (1:2), gave the title acetate as a foam (80% yield), $\nu_{max}$. (film) 3385, 3260, 1777 and 1759(sh)cm$^{-1}$.

Preparation 9(d)

(3S,4R) 3-[Acetoxy(3-methyl-2-isoxazolin-5-yl)methyl]-1-(1-p-nitrobenzyloxycarbonyl-1-triphenylphosphoranylidenemethyl)-4-tritylthioazetidin-2-one

The acetate from Preparation 9(c) was dissolved in benzene together with p-nitrobenzyl glyoxylate monohydrate. The mixture was heated to reflux using Dean and Stark apparatus. After 20 minutes the benzene was distilled off until the volume was reduced to 10ml and cooled. Finally the reaction was treated with one drop of triethylamine and thence treated, as for Preparation 1(e), with thionyl chloride and triphenylphosphine. After reacting with triphenylphosphine for 16 hours the reaction was diluted with ethyl acetate and washed with citric acid. Evaporation and chromatography on silica, eluting with ethyl acetate/n-hexane (8:3), gave the title phosphorane (72% yield), $\nu_{max}$.(film) 1753, 1645 and 1625cm$^{-1}$.

Preparation 9(e)

(5R,6S) p-Nitrobenzyl 6-[Acetoxy(3-methyl-2-isoxazolin-5-yl)methyl]penem-3-carboxylate

The phosphorane from Preparation 9(d) was dissolved in methanol and treated with silver nitrate as described in Preparation 1(f). After 15 minutes the reaction was evaporated, redissolved in dichloromethane and treated with formic acetic anhydride in accordance with Preparation 1(g). The final solution was warmed to 40$^{o}$C for 3 hours before being evaporated and chromatographed on silica, eluting with ethyl acetate/n-hexane (2:1), to give the title penem as a foam (90% yield), $\nu_{max}$ (KBr) 1791, 1749 and 1720cm$^{-1}$.

Example 9(a)

(5R) p-Nitrobenzyl (Z)-6-[(3-Methyl-2-isoxazolin-5-yl) methylene]penem-3-carboxylate

The penem from Preparation 9(e) was dissolved in dichloromethane and treated with DBU as for Example 1(a). Similar work up and chromatography on silica, eluting with ethyl acetate/n-hexane (3:1), gave the title penem as a solid from diethylether (49% yield), M.p. 75-79°C; $[\alpha]_D^{23}$ 0° (c0.175, $CHCl_3$); $\nu_{max}$ (KBr) 1784 and 1716cm$^{-1}$.

Example 9(b)

(5R) Sodium (Z)-6-[(3-Methyl-2-isoxazolin-5-yl) methylene]penem-3-carboxylate

The penem from Example 9(a) was hydrogenolysed in accordance with Example 1(b) for 20 minutes. Chromatography on Biogel P2 and freeze-drying of the appropriate fractions gave the title salt (36% yield), $[\alpha]_D^{23}$ -56°(c0.1866, $H_2O$); $\nu_{max}$ (KBr) 1763 and 1616cm$^{-1}$; $\delta(D_2O)$ 2.06 (3H, s), 3.05 and 3.47 (2H, ABX, J6.7, 11.5 and 17.7Hz), 5.4-5.55 (1H, m), 6.51 (1H, d, J3.5Hz), 6.56 (1H, d, J 0.7Hz), 7.12 (1H, s).

Preparation 10(a)

(3S,4R) 3-[Hydroxy(3-methyl-2-isoxazolin-5-yl) methyl]-4-tritylthioazetidin-2-one

The more polar isomer (isomer K2) from Preparation 9(a) was reduced with sodium borohydride and deprotected with potassium fluoride in accordance with the procedure set out in Preparation 9(b). Identical work

up and purification gave the title alcohol as a foam (54% yield), $\nu_{max}$ (film) 3390 and 1760cm$^{-1}$.

Preparation 10(b)

(3S,4R) 3-[Acetoxy(3-methyl-2-isoxazolin-5-yl)methyl] -4-tritylthioazetidin-2-one

The alcohol from Preparation 10(a) was treated as for the isomer K1 in accordance with Preparation 9(c). The title acetate was isolated as a foam (62% yield), $\nu_{max}$ (film) 3385, 3290, 1777 and 1740cm$^{-1}$.

Preparation 10(c)

(3S,4R) 3-[Acetoxy(3-methyl-2-isoxazolin-5-yl)methyl] -1-(1-p-nitrobenzyloxycarbonyl-1-triphenylphosphoranylidenemethyl)-4-tritylthioazetidin-2-one

The acetate from Preparation 10(b) was treated as for the isomer K1 in accordance with Preparation 9(d). The title phosphorane was isolated as a foam (75% yield), $\nu_{max}$ (film) 1750, 1645 and 1622cm$^{-1}$.

Preparation 10(d)

(5R,6S) p-Nitrobenzyl 6-[Acetoxy(3-methyl -2-isoxazolin-5-yl)methyl]penem-3-carboxylate

The phosphorane from Preparation 10(c) was treated as for the isomer K1 in accordance with Preparation 9(e). The title penem was isolated as a foam (58% yield), $\nu_{max}$(KBr) 1792, 1746 and 1720 cm$^{-1}$.

Example 10(a)

(5R) p-Nitrobenzyl (Z)-6-[(3-Methyl-2-isoxazolin-5-yl)methylene]penem-3-carboxylate

The penem from Preparation 10(d) was treated as for the isomer K1 in accordance with Example 9(a). The title penem was isolated as a crystalline solid from ethyl acetate/n-hexane (58% yield), M.p.137-139°C;$[\alpha]_D^{23}$ + 129°(c0.1366, $CHCl_3$); $\nu_{max}$ (KBr) 1791 and 1711cm$^{-1}$; $\delta(CDCl_3)$ 2.05 (3H, s), 2.82 and 3.31 (2H, ABMX, J 0.6, 1, 6, 11.3 and 16.9Hz), 5.2-5.49 (3H, m), 6.29 (1H, dd, J 1 and 3.1Hz), 6.47 (1H, dd, J 0.7 and 1Hz), 7.40 (1H, s) and 7.52-8.29 (4H, aromatics).

Example 10(b)

(5R) Sodium (Z)-6-[(3-Methyl-2-isoxazolin-5-yl)methylene]penem-3-carboxylate

The penem from Example 10(a) was treated as for the isomer K1 in accordance with Example 9(b). The title penem was thus isolated as a freeze-dried solid (56% yield), $[\alpha]_D^{23}$ + 160° (C 0.1116, $H_2O$); $\nu_{max}$ (KBr) 1763 and 1607cm$^{-1}$; $\delta(D_2O)$ 2.07 (3H, s), 3.08 and 3.48 (2H, ABX, J 5.9, 11.3 and 17.6Hz), 5.36-5.48 (1H, m), 6.42-6.53 (2H, m) and 7.13 (1H, s).

Preparation 11(a)

(3RS,4SR) 1-t-Butyldimethylsilyl-3-[(5RS)1-methyl-2-oxopyrrolidin-5-ylcarbonyl]-4-tritylthioazetidin-2-one and (3RS,4SR) 1-t-Butyldimethylsilyl-3-[(5SR)1-methyl-2-oxopyrrolidin-5-ylcarbonyl]-4-tritylthioazetidin-2-one

The anion of (4RS) 1-t-butyldimethylsilyl-4-tritylthioazetidin-2-one was formed in accordance with the procedure delineated in Preparation 3(a). The anion

was quenched with ethyl (2RS) 1-methyl-5-oxo-pyrrolidine-2-carboxylate dissolved in tetrahydrofuran. After 15 minutes the reaction was further quenched with saturated aqueous ammonium chloride, then diluted with ethyl acetate. Following washes with saturated sodium hydrogen carbonate and brine solutions,the solvent was evaporated. Chromatography of the residue on silica, eluting with ethyl acetate, gave the title ketones as a homogenous crystalline solid from diethylether (77% yield), M.p. 173-179°C (dec); $\nu_{max}$ (KBr) 1751, and 1706cm$^{-1}$.

Preparation 11(b)

(3RS,4SR) 3-[Hydroxy((5RS)1-methyl-2-oxo-pyrrolidin-5-yl)methyl]-4-tritylthioazetidin-2-one and (3RS,4SR) 3-[Hydroxy((5SR)1-methyl-2-oxo-pyrrolidin-5-yl)methyl] -4-tritylthioazetidin-2-one

The ketones from Preparation 11(a) were dissolved in dimethoxyethane/tetrahydrofuran/ethanol (10:5:1) and treated with sodium borohydride in accordance with Preparation 3(b). After 1 hour the reaction was diluted with ethyl acetate and washed with dilute citric acid, brine and finally with an aqueous saturated sodium hydrogen carbonate solution. The ethyl acetate was evaporated and the residue redissolved in dimethoxyethane/methanol (6:5). This solution was treated with potassium fluoride as described in Preparation 1(c) and diluted with ethyl acetate after 15 minutes. After washing with brine (3 x), concentration of the solution by evaporation of the solvent caused the title alcohols to co-crystallize (70% yield), M.p. 204-205° (dec); $\nu_{max}$ (KBr) 3383, 3057, 3029, 1768 and 1669cm$^{-1}$.

Preparation 11(c)

(3RS,4SR)3-[Acetoxy((5RS)1-methyl-2-oxo-pyrrolidin-5-yl)methyl]-4-tritylthioazetidin-2-one and (3RS,4SR) 3-[Acetoxy((5SR)1-methyl-2-oxo-pyrrolidin-5-yl)methyl] -4-tritylthioazetidin-2-one

The alcohols from Preparation 11(b) were dissolved in dichloromethane and reacted with acetic anhydride in accordance with Preparation 1(d). After 30 minutes the reaction was diluted with ethyl acetate and washed with brine, dilute citric acid and finally with a saturated sodium hydrogen carbonate solution. Chromatography on silica, eluting with ethyl acetate, gave the title acetates as a homogenous gum (51% yield), $\nu_{max}$ (KBr) 3386, 3219, 1774, 1745 and 1688cm$^{-1}$.

Preparation 11(d)

(3RS,4SR) 3-[Acetoxy((5RS)1-methyl-2-oxo-pyrrolidin -5-yl)methyl]-1-(1-p-nitrobenzyloxycarbonyl-1-triphenyl-phosphoranylidenemethyl)-4-tritylthioazetidin -2-one and (3RS,4SR)3-[Acetoxy((5SR)1-methyl-2-oxo-pyrrolidin-5-yl)methyl]-1-(1-p-nitrobenzyloxycarbonyl -1-triphenylphosphoranylidenemethyl)-4-tritylthio-azetidin-2-one

The acetates from Preparation 11(c) were reacted sequentially with p-nitrobenzyl glyoxylate monohydrate/triethylamine, 2,6-lutidine/thionyl chloride and finally with triphenylphosphine/ 2,6-lutidine in accordance with Preparation 1(f). Purification of the product by chromatography on silica, eluting with ethyl acetate, gave the title phosphoranes as a homogenous gum (50% yield), $\nu_{max}$ (film) 1759, 1692, 1657 and 1629cm$^{-1}$.

Preparation 11(e)

(5RS,6SR) p-Nitrobenzyl 6-[Acetoxy((5RS)1-methyl-2-oxo-pyrrolidin-5-yl)methyl]penem-3-carboxylate and (5RS,6SR) p-Nitrobenzyl 6-[Acetoxy((5SR)1-methyl-2-oxo-pyrrolidin-5-yl)methyl]penem-3-carboxylate

The phosphoranes from Preparation 11(d) were dissolved in dichloromethane/methanol (3:4) and treated sequentially with pyridine and silver nitrate in accordance with Preparation 1(f). After 20 minutes the reaction was evaporated and redissolved in dichloromethane. Formic acetic anhydride/4-dimethylaminopyridine and then triethylamine hydrochloride were added as for Preparation 1(g). After 30 minutes the reaction was filtered, diluted with chloroform, and washed sequentially with brine, dilute citric acid and aqueous sodium hydrogen carbonate. The solution was warmed to 50°C for 15 minutes and then evaporated. The title penems were isolated by crystallization of the residue from ethyl acetate/diethylether (51% yield), $\nu_{max.}$ (KBr) 1787, 1749, 1710 and 1685cm$^{-1}$.

Example 11(a)

(5RS) p-Nitrobenzyl 6-[((5RS)1-Methyl-2-oxo-pyrrolidin-5-yl)methylene]penem-3-carboxylate and (5RS)p-Nitrobenzyl 6-[((5SR)1-Methyl-2-oxo-pyrrolidin-5-yl)methylene]penem-3-carboxylate

The penems from Preparation 11(e) were dissolved in dichloromethane and treated with DBU in accordance with Example 1(a). After 25 minutes the cooling bath was removed and stirring continued for a further 10 minutes. Dilution with ethyl acetate and sequential washes with citric acid, brine and aqueous sodium hydrogen carbonate were followed by evaporation at

reduced pressure. Chromatography on silica, eluting with ethyl acetate, gave the title penems as a foam (65% yield), $\nu_{max}$ (KBr) 1782, 1715, and 1686$cm^{-1}$.

Example 11(b)

(5RS) Sodium (Z)-6-[((5RS)1-Methyl-2-oxo-pyrrolidin-5-yl)methylene]penem-3-carboxylate and (5RS) Sodium (Z)-6-[((5SR)1-Methyl-2-oxo-pyrrolidin-5-yl)methylene]penem-3-carboxylate

The penems from Example 11(a) were dissolved in dioxane/water (3:1) and hydrogenated in the presence of 10% palladium/charcoal in accordance with Example 1(b). After 25 minutes the reaction was filtered and an equal volume of water, containing one equivalent of sodium hydrogen carbonate, was added. The reaction was stirred for 20 minutes before being concentrated and passed through a column of Biogel P2 eluting with water. The appropriate fractions were combined and freeze-dried to give the title salts as a 1:1 mixture (76%), $\nu_{max}$ (KBr) 1763, 1676 and 1609$cm^{-1}$; $\delta(D_2O)$ 1.84-2.03 and 2.32-2.64 (8H, m), 4.4-4.53 (1H, m), 4.86-5.02 (1H, m), 6.12 (1H, d, $J$ 9.5Hz), 6.40 (1H, s), 6.49 (1H, d, $J$ 9.1Hz), 6.54 (1H, d, $J$ 0.9Hz), 7.15 (1H, s) and 7.17 (1H, s).

Preparation 12(a)

(3S,4R) 1-t-Butyldimethylsilyl-3-(2,3-epoxy-1-hydroxybutyl)-4-tritylthioazetidin-2-one

The more polar isomer of (3S,4R) 1-t-butyldimethylsilyl-3-(2,3-epoxybutyryl)-4-tritylthioazetidin-2-one (designated K2) formed in Preparation 5(a), was reduced with sodium borohydride and worked up as for isomer K1. Thus the title epoxide (K2 isomer) was obtained as a foam (28% yield), $\nu_{max}$ (film) 3420 and 1740$cm^{-1}$.

Preparation 12(b)

(3S,4R) 3-(2,3-Epoxy-1-hydroxybutyl)-4-tritylthioazetidin-2-one

The alcohol from Preparation 12(a)(isomer K2) was reacted and worked up as is described for isomer K1 in Preparation 5(b). The title alcohol was thus obtained as a foam (89% yield), $\nu_{max}$ (film) 3385 and 1764cm$^{-1}$.

Preparation 12(c)

(3S,4R) 3-(1-Acetoxy-2,3-epoxybutyl)-4-tritylthio azetidin-2-one

The alcohol from Preparation 12(b)(isomer K2) was acetylated in accordance with Preparation 5(c). The reaction was diluted with ethyl acetate and washed sequentially with brine, dilute citric acid and finally aqueous sodium hydrogen carbonate solution. After evaporation of solvent at reduced pressure, the residue was chromatographed, eluting with ethyl acetate/n-hexane (1:1), giving the title acetate as a foam (94% yield), $\nu_{max}$ (film) 3380, 3295, 1774 and 1750 (sh)cm$^{-1}$.

Preparation 12(d)

(3S,4R) 3-(1-Acetoxy-2,3-epoxybutyl)-1-(1-p-nitro benzyloxycarbonyl-1-triphenylphosphoranylidenemethyl) -4-tritylthioazetidin-2-one

The acetate from Preparation 12(c)(isomer K2) was treated sequentially with p-nitrobenzyl glyoxalate monohydrate/triethylamine, thionyl chloride/2,6-lutidine and finally with triphenylphosphine/2,6-lutidine in accordance with Preparation 5(d). The title phosphorane was isolated as a foam (74% yield), 1752, 1649 and 1624cm$^{-1}$.

Preparation 12(e)

(5R,6S) p-Nitrobenzyl 6-(1-Acetoxy-2,3-epoxybutyl) penem-3-carboxylate

The phosphorane from Preparation 12(d)(isomer K2) was reacted sequentially with silver nitrate/pyridine and formic acetic anhydride/4-dimethylaminopyridine/ triethylamine hydrochloride, in the manner described for isomer K1 in Preparation 5(e). The title penem was thus obtained as a foam (65% yield), $\nu_{max}$ (film) 1788, 1749 and 1726cm$^{-1}$.

Example 12(a)

(5R) p-Nitrobenzyl (Z)-6-(2,3-Epoxybutylidene)penem -3-carboxylate

The penem from Preparation 12(e)(isomer K2) was treated with DBU as for isomer K1, in accordance with Example 5(a). The title penem was isolated by crystallization from ethyl acetate (48% yield), $\nu_{max}$.(film) 1790 and 1722cm$^{-1}$.

Example 12(b)

(5R) Sodium (Z)-6-(2,3-Epoxybutylidene)penem -3-carboxylate

The penem from Example 12(a)(isomer K2) was hydrogenated and purified as described for the K1 isomer in Example 5(b). The title penem was thus obtained as a freeze-dried solid (11%yield), $\delta(D_2O)$ 1.39(3H, d, *J* 5.2Hz), 3.20-3.29(1H, m), 3.50(1H, dd, *J* 2.2 and 4.8Hz), 6.44 (1H, s), 6.47 (1H, dd, *J* 0.6 and 5Hz)and 7.1 (1H, s).

Biological data

The following table summarises the β-lactamase activity of selected compounds according to the invention, as identified by Example numbers, against selected micro-organisms. The data is given in the form of the minimum inhibitory concentration (MIC) of amoxycillin in μg/ml when used in conjunction with 5μg/ml of the respective compound according to the invention. The MIC values for amoxycillin alone against the same organisms, and also the MIC values for the compounds according to the invention alone (given in brackets), are given for comparison purposes. In the Table 'NT' means 'not tested'.

TABLE

Amoxycillin MIC values (μg/ml) in the presence of 5μg/ml of compounds of this invention

| Organism | Amoxycillin alone | Amoxycillin plus compound of Example No.: (Compound alone) | | | | | |
|---|---|---|---|---|---|---|---|
| | | 1b | 2b | 3b | 5b | 8b | 12b |
| E.coli JT39 | >1000 | 2 (128) | 2 (>512) | 2 (512) | 2 (64) | 8 (512) | 2 (NT) |
| P.mirabilis C889 | >1000 | 2 (>512) | 32 (512) | 8 (>512) | 16 (256) | 128 (512) | 4 (NT) |
| K.pneumoniae E70 | 250 | 2 (>512) | 4 (>512) | 4 (>512) | 4 (128) | 16 (>512) | 2 (NT) |
| K.pneumoniae Ba95 | >1000 | 4 (256) | 8 (>512) | 8 (512) | 4 (512) | 16 (>512) | 2 (NT) |
| E.cloacae N1 | 500 | 2 (512) | 4 (512) | 8 (512) | 4 (512) | 8 (>512) | 4 (NT) |
| P.aeruginosa A | >1000 | 256 (>512) | >512 (>512) | >512 (>512) | 512 (>512) | >512 (>512) | 512 (NT) |
| S.aureus Russell | 100 | 0.13 (16) | 0.25 (32) | 0.13 (32) | 0.13 (8) | NT (NT) | 0.13 (NT) |

Claims

1. A compound of the general formula I:

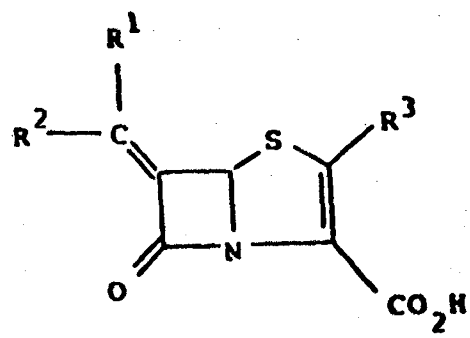

I

or a pharmaceutically acceptable salt or in vivo hydrolysable ester thereof

in which

one or $R^1$ and $R^2$ denotes hydrogen,

the other of $R^1$ and $R^2$ denotes an unsubstituted or substituted non-aromatic heterocyclyl group bonded through a carbon atom thereof, and

$R^3$ denotes hydrogen or an organic group.

2. A compound as claimed in claim 1, which is of the general formula IA:

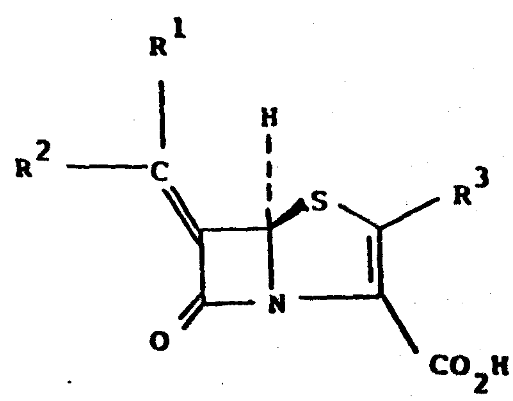

IA

in which $R^1$, $R^2$ and $R^3$ are defined as in claim 1.

3. A compound as claimed in claim 1 or claim 2, wherein one of $R^1$ and $R^2$ denotes hydrogen, and the other of $R^1$ and $R^2$ denotes a group selected from:

(i) a three-membered saturated heterocyclyl group containing one heteroatom selected from oxygen, nitrogen and sulphur, and being unsubstituted or being substituted by up to three monovalent substituents or one divalent substituent;

(ii) a four-membered heterocyclyl group containing one or two heteroatoms selected from oxygen, nitrogen and sulphur (with the proviso that the ring does not contain two adjacent oxygen atoms), and being saturated or containing one ring double bond, and being unsubstituted or substituted by up to five monovalent substituents and/or up to two divalent substituents;

(iii) a five-membered non-aromatic heterocyclyl group containing one, two or three heteroatoms selected from oxygen, nitrogen and sulphur (with the proviso that the ring does not contain two adjacent oxygen atoms), and being saturated or containing one ring double bond, and being unsubstituted or substituted by up to seven monovalent substituents and/or up to three divalent substituents; and

(iv) a six-membered non-aromatic heterocyclyl group containing from one to four heteroatoms selected from oxygen, nitrogen and sulphur (with the

proviso that the ring does not contain two adjacent oxygen atoms), and being saturated or containing one or two ring double bonds, and being unsubstituted or substituted by up to nine monovalent substituents and/or by up to three divalent substituents.

4. A compound as claimed in any one of claims 1 to 3, wherein $R^1$ denotes a hydrogen atom and $R^2$ denotes the non-aromatic heterocyclyl group.

5. A compound as claimed in claim 1, which is selected from:

(5R) (Z)-6-[(1,3-dioxolan-4-yl)methylene]penem-3-carboxylic acid;

(5RS) (Z)-6-(2,3-epoxybutylidene)penem-3-carboxylic acid;

(5R) (Z)-6-(2,3-epoxybutylidene)penem-3-carboxylic acid;

(5RS) (Z)-6-[(N-benzyloxycarbonyl-pyrrolidin-2-yl) methylene]penem-3-carboxylic acid;

(5R) (Z)-6-([(2S)-azetidin-2-yl]methylene)penem-3-carboxylic acid;

(5RS) (Z)-6-[(3,4-dihydro-2H-pyran-2-yl)methylene]-penem-3-carboxylic acid;

(5R) (E)-6-([(4R)-1-methyloxy-2-oxoazetidin-4-yl]-methylene)penem-3-carboxylic acid;

(5R) (Z)-6-[(3-methyl-2-isoxazolin-5-yl)methylene]-penem-3-carboxylic acid;

(5RS) (Z)-6-[(1-methyl-2-oxo-pyrrolidin-5-yl)methylene] penem-3-carboxylic acid;

and pharmaceutically acceptable salts and in-vivo hydrolysable esters thereof.

6. A process for the preparation of a compound of the general formula I as defined in claim 1, or a salt or ester thereof, which comprises eliminating the elements of a compound of the general formula XIII:

$$H-X^{o} \qquad XIII$$

from a penem or penem intermediate of the general part-formula XIV:

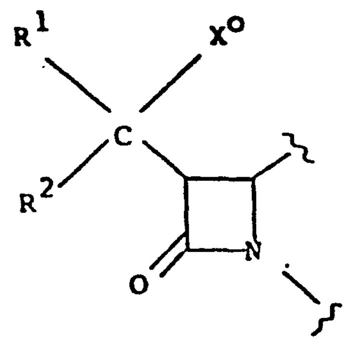

XIV

in which

$R^1$ and $R^2$ are defined as in claim 1, and

$X^o$ denotes a hydroxy group or a leaving group,

to give a compound of the general part-formula XV:

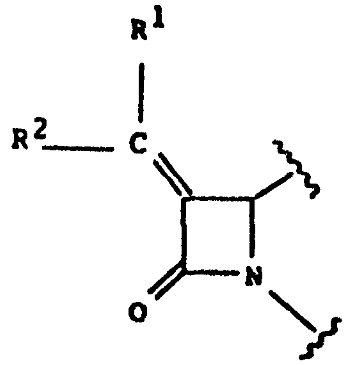

XV

in which $R^1$ and $R^2$ are defined as in claim 1,

and, if the resulting compound of the general formula XV is a penem intermediate, converting it into a penem of the general formula I or a salt or ester thereof.

7. A pharmaceutical composition which comprises a compound as claimed in any one of claims 1 to 5, in admixture or conjunction with a pharmaceutically acceptable carrier.

8. A pharmaceutical composition as claimed in claim 7, which additionally comprises a penicillin, cephalosporin or other β-lactam antibiotic.

European Patent Office

**EUROPEAN SEARCH REPORT**

0210065

Application number

EP 86 30 5584

**DOCUMENTS CONSIDERED TO BE RELEVANT**

| Category | Citation of document with indication, where appropriate, of relevant passages | Relevant to claim | CLASSIFICATION OF THE APPLICATION (Int Cl 4) |
|---|---|---|---|
| D,A | EP-A-0 041 768 (BEECHAM) * Claims * ----- | 1,6-8 | C 07 D 499/00<br>A 61 K 31/43 //<br>C 07 F 7/10<br>C 07 F 9/65<br>C 07 D 405/06<br>C 07 D 403/06<br>C 07 D 413/06<br>C 07 D 205/08 |
| | | | TECHNICAL FIELDS SEARCHED (Int Cl 4) |
| | | | C 07 D 499/00<br>A 61 K 31/00 |

The present search report has been drawn up for all claims

| Place of search | Date of completion of the search | Examiner |
|---|---|---|
| THE HAGUE | 18-09-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82